# EUROPEAN PATENT APPLICATION

(11) **EP 4 699 610 A1**
(43) Date of publication of application: **25.02.2026**
(21) Application number: 24792763.5
(22) Date of filing: 19.04.2024
(51) Int. Cl.: A61K 31/675, A61K 45/00, A61P 35/00, A61P 43/00

(54) **PHARMACEUTICAL COMPOSITION FOR TREATING GLIOMA**

(30) Priority: 21.04.2023 JP 2023070183
(71) Applicant: SymBio Pharmaceuticals Limited, Tokyo 105-0001 (JP)
(72) Inventor: HAZAMA, Masatoshi, Tokyo 105-0001 (JP); NAGATANI, Shoji, Tokyo 105-0001 (JP)
(74) Representative: Gulde & Partner
(86) International application number: PCT/JP2024/015580
(87) International publication number: WO 2024/219491

(57) **Abstract**

A method for treating glioma is provided. A pharmaceutical composition for treatment of glioma, comprising brincidofovir, a pharmaceutically acceptable salt thereof, or a solvate thereof, is used. A pharmaceutical composition or a kit, comprising brincidofovir, a pharmaceutically acceptable salt thereof, or a solvate thereof, and a chemotherapeutic agent, may also be used.

## Description

### Technical Field

The technical field of the present invention relates to treatment of glioma.

### Background art

A brain tumor is a general term for a tumor that occurs in the cranial cavity. Non-Patent Literature 1 describes in its abstract that brain and other central nervous system tumors are among the most fatal cancers and account for substantial morbidity and mortality in the United States. Glioma is a type of malignant brain tumor. Non-Patent Literature 2 describes in its abstract that despite great strides made in understanding the pathophysiology of high-grade gliomas over the past two decades, most patients succumb to these neoplasias within two years of diagnosis.

Among gliomas, glioblastoma is known as a highly aggressive brain tumor. Non-Patent Literature 3 describes in its abstract that glioblastoma is the most common malignant primary brain tumor in adults and is almost invariably fatal, and that despite a growing understanding of the various mechanisms underlying treatment failure, the standard-of-care therapy has not changed over the last two decades, signifying a great unmet need. In addition, the introduction of Non-Patent Literature 3 describes that, looking back at past clinical trials, there are currently no effective therapies for glioblastoma. Non-Patent Literature 4 describes diagnostic methods and prognosis for glioblastoma. Furthermore, the introduction of Non-Patent Literature 4 describes that glioblastoma is characterized by poor prognosis, low survival rates, and extremely limited opportunities for therapy.

### Citation List

### Non-Patent Literature

Non-Patent Literature 1: "Brain and other central nervous system tumor statistics, 2021" Miller et al., CA Cancer J Clin. 2021 Sep;71(5):381-406.
Non-Patent Literature 2: "Interactions between Tumor Cells, Neurons, and Microglia in the Glioma Microenvironment" Radin et al., Int J Mol Sci. 2020 Nov 11;21(22):8476.
Non-Patent Literature 3: "Molecular Mechanisms of Treatment Resistance in Glioblastoma" Ou et al., Int J Mol Sci. 2020 Dec 31;22(1):351.
Non-Patent Literature 4: "Current and Future Trends on Diagnosis and Prognosis of Glioblastoma: From Molecular Biology to Proteomics" Silantyev et al., Cells. 2019 Aug 9;8(8):863.

### Summary of Invention

### Technical Problem

Despite various findings accumulated thus far, the mechanism of glioma is still unknown in many respects, and thus, conventional therapeutic approaches alone have not been sufficient.

On the other hand, as a result of diligent research, the present inventors have discovered that brincidofovir demonstrates excellent efficacy in the treatment of glioblastoma.

### Solution to Problem

According to one aspect of the present invention, a pharmaceutical composition for treatment of glioma, comprising brincidofovir, a pharmaceutically acceptable salt thereof, or a solvate thereof is provided. This composition can be used to treat glioma.

According to one aspect of the present invention, a pharmaceutical composition or a kit, comprising brincidofovir, a pharmaceutically acceptable salt thereof, or a solvate thereof, and an alkylating agent, a pharmaceutically acceptable salt thereof, or a solvate thereof is provided. This composition or kit can be used to treat glioma.

### Brief Description of Drawings

[Figure 1] Fig. 1 shows the results of an examination of glioblastoma cell proliferation in the brains of glioblastoma animal models.
[Figure 2] Fig. 2 shows the results of an examination of the survival rate of glioblastoma animal models.
[Figure 3] Fig. 3 shows the results of an examination of glioblastoma cell proliferation in the brains of glioblastoma animal models.
[Figure 4] Fig. 4 shows the results of an examination of glioblastoma cell proliferation in the brains of glioblastoma animal models.
[Figure 5] Fig. 5 shows the results of an examination of pharmacokinetic parameters in mice after BCV administration.
[Figure 6] Fig. 6 shows pharmacokinetic parameters in humans after BCV administration.
[Figure 7] Fig. 7 shows the results of an examination of concentration-cytotoxicity curves and respective IC₅₀ values of BCV for various human glioblastoma cell lines.
[Figure 8] Fig. 8 shows the results of an examination of concentration-cytotoxicity curves and respective IC₅₀ values of BCV for various human glioblastoma cell lines.
[Figure 9] Fig. 9 shows the results of an examination of MGMT protein expression in U-87MG/MGMT pooled cells by Western blotting.
[Figure 10] Fig. 10 shows the results of an examination of MGMT protein expression in U-87MG/MGMT monoclonal cells by Western blotting.
[Figure 11] Fig. 11 shows the results of an examination of concentration-cytotoxicity curves and respective IC₅₀ values of BCV for U-87MG/MGMT monoclonal cells.

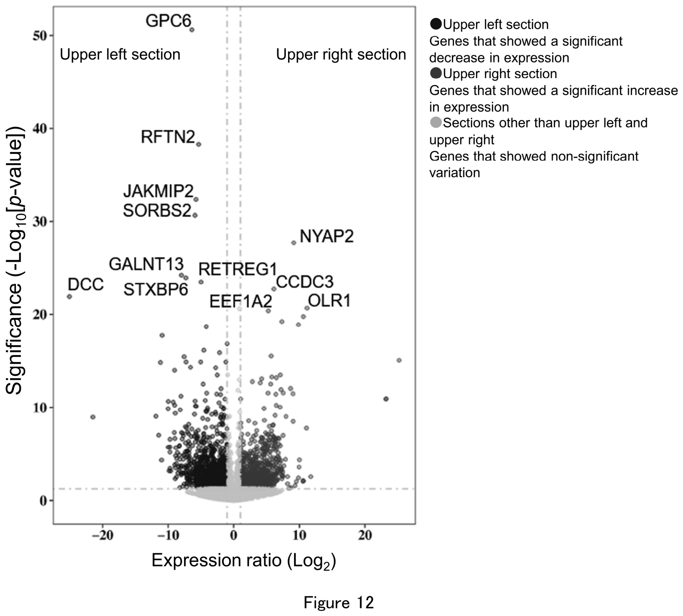

[Figure 12] Fig. 12 shows a volcano plot of the top three BCV-highly sensitive human glioblastoma cell line groups versus the remaining eight cell line groups. The plot in Fig. 12 shows the relative relationship when comparing the respective cell line groups. Fig. 12 is divided into six sections by dotted lines. The circles in the upper left section, which includes GPC6 and the like, indicate genes that showed a significant decrease in expression in the highly sensitive group. The circles in the upper right section, which includes NYAP2 and the like, indicate genes that showed a significant increase in expression in the highly sensitive group. The other four sections contain genes with a relatively weak association, where the expression difference between the sensitive cell line group and the remaining cell line group is 2-fold or less, or the significance of the association between the sensitive cell line group and the remaining cell line group is 0.01 or more.
[Figure 13] Fig. 13 shows the results of an examination of the NYAP2 gene expression level (nTPM) and IC₅₀ values for BCV in 80 human brain tumor cell lines.
[Figure 14] Fig. 14 shows the results of an examination of the difference in NYAP2 gene expression level (nTPM) between the three BCV-highly sensitive cell lines and the remaining eight cell lines.
[Figure 15] Fig. 15 shows the results of an examination of the GPC6 gene expression level (nTPM) and IC₅₀ values for BCV in 80 human brain tumor cell lines.
[Figure 16] Fig. 16 shows the results of an examination of the difference in GPC6 gene expression level (nTPM) between the BCV-highly sensitive cell lines and the remaining eight cell lines.
[Figure 17] Fig. 17 shows the results of an examination of glioblastoma cell proliferation in the brains of glioblastoma animal models.
[Figure 18] Fig. 18 shows a Kaplan-Meier curve plotting the cumulative survival rate after implantation of human brain tumor cells.
[Figure 19] Fig. 19 shows the main genomic characteristics of seven patient-derived brain tumor cells. In the figure, "amp" means amplification, "wt" means wild type, and "mutant" means mutant type.

### Description of Embodiments

Hereinafter, embodiments of the present invention will be described in detail. Note that repeated descriptions of similar content are omitted where appropriate in order to avoid redundancy.

### (1) Method

An embodiment of the present invention provides a method for treating glioma, comprising the step of administering brincidofovir (BCV), a pharmaceutically acceptable salt thereof, or a solvate thereof to a subject. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, it is possible to treat glioblastoma, which is a highly malignant type of glioma and is generally difficult to treat. Using this method, for example, the life of a subject with glioma can be prolonged with a dose suitable for clinical application or a low dose of the agent.

### (2) Method

An embodiment of the present invention provides a method for inhibiting the proliferation of glioma cells, comprising the step of administering BCV, a pharmaceutically acceptable salt thereof, or a solvate thereof to a subject. This method can be used to inhibit the proliferation of glioma cells through a novel therapeutic approach.

### (3) Method

An embodiment of the present invention provides a method for prolonging life, comprising the step of administering BCV, a pharmaceutically acceptable salt thereof, or a solvate thereof to a subject. This method can be used to prolong the life of a subject through a novel therapeutic approach.

### (4) Method

An embodiment of the present invention provides a method for treating glioma, in a combination treatment of BCV, a pharmaceutically acceptable salt thereof, or a solvate thereof, and a chemotherapeutic agent, the method comprising the step of administering BCV, a pharmaceutically acceptable salt thereof or a solvate thereof, or the chemotherapeutic agent to a subject. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, the life of a subject with glioma can be prolonged with a dose suitable for clinical application or a low dose of the agent. Using this method, for example, a synergistic or additive effect of BCV and the chemotherapeutic agent can be obtained. The chemotherapeutic agent is preferably temozolomide (TMZ), a pharmaceutically acceptable salt thereof, or a solvate thereof, from the viewpoint that a significant therapeutic effect is exerted in combination with a lower dose of BCV. Herein, TMZ is an abbreviation for temozolomide, and they have the same meaning.

### (5) Method

An embodiment of the present invention provides a method for treating glioma, comprising the step of administering BCV, a pharmaceutically acceptable salt thereof, or a solvate thereof to a subject, wherein the subject is a subject to whom a chemotherapeutic agent is administered prior to, concurrently with, or subsequent to the administration of BCV, a pharmaceutically acceptable salt thereof, or a solvate thereof. Using this method, the same effect as in the method of (4) above can be obtained. Concurrent administration includes administration within the same time period and any administration performed substantially concurrently, taking usual treatment procedures into account. Concurrent administration includes administration of BCV and a chemotherapeutic agent as a combination preparation. From another aspect, an embodiment of the present invention provides a method for treating glioma, comprising the step of administering a chemotherapeutic agent to a subject, wherein the subject is a subject to whom BCV, a pharmaceutically acceptable salt thereof, or a solvate thereof is administered prior to, concurrently with, or subsequent to the administration of the chemotherapeutic agent. From another aspect, an embodiment of the present invention provides a method for treating glioma, comprising the step of administering BCV, a pharmaceutically acceptable salt thereof, or a solvate thereof to a subject to whom a chemotherapeutic agent has been administered. From another aspect, an embodiment of the present invention provides a method for treating glioma, comprising the step of administering a chemotherapeutic agent to a subject to whom BCV, a pharmaceutically acceptable salt thereof, or a solvate thereof has been administered. From another aspect, an embodiment of the present invention provides a method for treating glioma, comprising the step of administering BCV, a pharmaceutically acceptable salt thereof, or a solvate thereof to a subject, and the step of administering a chemotherapeutic agent to the subject. From another aspect, an embodiment of the present invention provides a method for treating glioma, comprising the step of administering BCV, a pharmaceutically acceptable salt thereof, or a solvate thereof, and a chemotherapeutic agent in combination to a subject.

### (6) Method

An embodiment of the present invention provides a method for treating glioma, in a combination treatment of administration of BCV, a pharmaceutically acceptable salt thereof, or a solvate thereof, and radiation therapy, the method comprising the step of administering BCV, a pharmaceutically acceptable salt thereof or a solvate thereof to a subject, or the step of performing radiation therapy on the subject. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, the life of a subject with glioma can be prolonged with a dose suitable for clinical application or a low dose of the agent. Using this method, for example, a synergistic or additive effect of BCV and radiation therapy can be obtained. From another aspect, an embodiment of the present invention provides a method for treating glioma, comprising the step of administering BCV, a pharmaceutically acceptable salt thereof, or a solvate thereof to a subject, wherein the subject is a subject on whom radiation therapy is performed prior to, concurrently with, or subsequent to the administration of BCV, a pharmaceutically acceptable salt thereof, or a solvate thereof. From another aspect, an embodiment of the present invention provides a method for treating glioma, comprising the step of performing radiation therapy on a subject, wherein the subject is a subject to whom BCV, a pharmaceutically acceptable salt thereof, or a solvate thereof is administered prior to, concurrently with, or subsequent to the radiation therapy treatment.

### (7) Method

An embodiment of the present invention provides a method for treating glioma, in a combination treatment of (a) administration of BCV, a pharmaceutically acceptable salt thereof, or a solvate thereof, (b) administration of a chemotherapeutic agent, and (c) radiation therapy, the method comprising the step of administering BCV, a pharmaceutically acceptable salt thereof or a solvate thereof to a subject, the step of administering a chemotherapeutic agent to the subject, or the step of performing radiation therapy on the subject. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, the life of a subject with glioma can be prolonged with a dose suitable for clinical application or a low dose of the agent. Using this method, for example, a synergistic or additive effect of BCV, the chemotherapeutic agent, and radiation therapy can be obtained. From another aspect, an embodiment of the present invention provides a method for treating glioma, comprising the step of administering BCV, a pharmaceutically acceptable salt thereof, or a solvate thereof to a subject, wherein the subject is a subject to whom a chemotherapeutic agent is administered and on whom radiation therapy is performed, prior to, concurrently with, or subsequent to the administration of BCV, a pharmaceutically acceptable salt thereof, or a solvate thereof. From another aspect, an embodiment of the present invention provides a method for treating glioma, comprising the step of administering a chemotherapeutic agent to a subject, wherein the subject is a subject to whom BCV, a pharmaceutically acceptable salt thereof, or a solvate thereof is administered and on whom radiation therapy is performed, prior to, concurrently with, or subsequent to the administration of the chemotherapeutic agent. From another aspect, an embodiment of the present invention provides a method for treating glioma, comprising the step of performing radiation therapy on a subject, wherein the subject is a subject to whom BCV, a pharmaceutically acceptable salt thereof, or a solvate thereof is administered and to whom a chemotherapeutic agent is administered, prior to, concurrently with, or subsequent to the radiation therapy treatment. From another aspect, an embodiment of the present invention provides a method for treating glioma, comprising the step of performing administration of BCV, a pharmaceutically acceptable salt thereof, or a solvate thereof, administration of a chemotherapeutic agent, and radiation therapy in combination on a subject.

### (8) Method

An embodiment of the present invention provides a method for treating glioma, comprising the step of administering BCV, a pharmaceutically acceptable salt thereof, or a solvate thereof to a human at a dose of 10-60 mg. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, it is possible to treat glioblastoma, which is a highly malignant type of glioma and is generally difficult to treat. Using this method, for example, the life of a subject with glioma can be prolonged with a dose suitable for clinical application or a low dose of the agent. The human includes an adult.

### (9) Method

An embodiment of the present invention provides a method for treating glioma, comprising the step of administering BCV, a pharmaceutically acceptable salt thereof, or a solvate thereof to a human at a dose of 40 mg. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, it is possible to treat glioblastoma, which is a highly malignant type of glioma and is generally difficult to treat. Using this method, for example, the life of a subject with glioma can be prolonged with a dose suitable for clinical application or a low dose of the agent. The human includes an adult.

### (10) Method

An embodiment of the present invention provides a method for treating glioma, in a combination treatment of BCV, a pharmaceutically acceptable salt thereof, or a solvate thereof, and a chemotherapeutic agent, the method comprising the step of administering BCV, a pharmaceutically acceptable salt thereof or a solvate thereof to a human at a dose of 20 mg. This method can be used to treat glioma through a novel therapeutic approach. Using this method, for example, a treatment with an excellent balance between therapeutic effect and safety can be performed. Using this method, for example, glioma can be treated with a dose suitable for clinical application (e.g., a dose with excellent efficacy and safety) or a low dose of the agent. Using this method, for example, the life of a subject with glioma can be prolonged with a dose suitable for clinical application or a low dose of the agent. Using this method, for example, a synergistic or additive effect of BCV and the chemotherapeutic agent can be obtained. The human includes an adult. The chemotherapeutic agent is preferably TMZ, a pharmaceutically acceptable salt thereof, or a solvate thereof, from the viewpoint that a significant therapeutic effect is exerted in combination with a lower dose of BCV.

### (11) Method

An embodiment of the present invention provides a method for treating glioma with high safety, comprising the step of administering BCV, a pharmaceutically acceptable salt thereof, or a solvate thereof to a subject. Here, high safety includes, for example, that substantially no systemic toxicity or substantially no nephrotoxicity is observed.

### (12) Method

An embodiment of the present invention provides a diagnostic method or a detection method, comprising the step of detecting the presence or absence of methylation of the promoter region of the MGMT gene of a subject. This method includes a diagnostic method or a detection method for evaluating, prior to treatment with BCV, a pharmaceutically acceptable salt thereof, or a solvate thereof, whether a subject has a glioma that has resistance to an alkylating agent (e.g., TMZ) or a glioma for which the therapeutic effect of an alkylating agent is low. By using this method, treatment can be provided to a population in which the therapeutic effect of an alkylating agent is low, or a population that is resistant to an alkylating agent. The detecting step may be performed before the treatment with BCV, a pharmaceutically acceptable salt thereof, or a solvate thereof is performed. The detecting step may be performed using methylation-specific PCR, unmethylation-specific PCR, or a pyrosequencing method. This method may include a step of collecting glioma cells from the subject, or a step of identifying a subject without the above-mentioned methylation as a subject to be treated. This method may be a method performed to identify a subject to be treated with BCV, a pharmaceutically acceptable salt thereof, or a solvate thereof. The above-mentioned subject includes a patient suffering from glioma or glioma cells of the patient. The diagnostic method includes a companion diagnostic method. From another aspect, an embodiment of the present invention provides a method for identifying a subject for administration of BCV, a pharmaceutically acceptable salt thereof, or a solvate thereof, the method comprising the step of detecting the presence or absence of methylation of the promoter region of the MGMT gene of the subject. From another aspect, an embodiment of the present invention provides a diagnostic method or a detection method, comprising the step of detecting the expression level of the MGMT gene of a subject. The step of detecting the expression level includes a step of detecting positive or negative. From another aspect, an embodiment of the present invention provides a kit or a composition for the diagnostic method or the detection method described above. This kit or composition includes, for example, a means for detecting methylation or unmethylation of the promoter region of the MGMT gene. The detection means may include, for example, a primer (e.g., a primer capable of amplifying at least a part of the promoter region (e.g., a primer that can bind to a site upstream or downstream of the promoter region)) or an antibody. The primer may be a methylation-specific or unmethylation-specific primer. The antibody may be an antibody specific to the promoter region of the MGMT gene in a methylated or unmethylated state, or a partial region thereof.

### (13) Method

An embodiment of the present invention provides a method for treating glioma, comprising the step of administering BCV, a pharmaceutically acceptable salt thereof, or a solvate thereof to a subject with glioma, wherein the subject is a subject identified based on the expression level of MGMT as an indicator. An embodiment of the present invention provides a method for treating glioma, comprising the step of administering BCV, a pharmaceutically acceptable salt thereof, or a solvate thereof to an MGMT-high-expressing-glioma subject. An embodiment of the present invention provides a method for detecting a high expression level of MGMT from glioma cells of a subject to be administered with BCV, a pharmaceutically acceptable salt thereof, or a solvate thereof, or from glioma cells of a subject for whom such administration is being considered. An embodiment of the present invention provides a method for selecting a treatment method or an active ingredient for use in treatment, the method comprising the step of using the expression level of MGMT as an indicator. An embodiment of the present invention provides a method for determining a subject for drug administration, the method comprising the step of testing the expression level of MGMT in a sample derived from a subject with glioma. An embodiment of the present invention provides a diagnostic method or a testing method, comprising the step of testing the expression level of MGMT of a subject. In these methods, the expression level of MGMT as a testing target or an indicator may be high expression or positive.

### (14) Method

An embodiment of the present invention provides a method for treating glioma, comprising the step of administering BCV, a pharmaceutically acceptable salt thereof, or a solvate thereof to a subject with glioma, wherein the subject is a subject identified based on the expression level of GPC6 or NYAP2 as an indicator. As described in the Examples below, the sensitivity to BCV in glioma was associated with the expression level of GPC6 or NYAP2. For example, it was shown that when the expression level of GPC6 is low, the inhibitory effect of BCV on glioma is high, and the sensitivity to BCV is high. For example, it was shown that when the expression level of NYAP2 is high, the inhibitory effect of BCV on glioma is high, and the sensitivity to BCV is high. Therefore, by using this method, sensitivity can be predicted based on the expression level of GPC6 or NYAP2 as an indicator, and more appropriate BCV treatment can be performed. Herein, a more appropriate BCV treatment includes, for example, a BCV treatment in which the probability of a therapeutic effect appearing is improved, or the therapeutic effect is improved, compared to the case of administration to an unspecified subject. For example, if the expression level of GPC6 of a subject is low and the sensitivity is predicted to be high, treatment may be performed by administering BCV or by administering it at a low dose. Also, for example, if the expression level of GPC6 of a subject is high and the sensitivity is predicted to be low, treatment may be performed by not administering BCV or by administering it at a high dose. For example, if the expression level of NYAP2 of a subject is high and the sensitivity is predicted to be high, treatment may be performed by administering BCV or by administering it at a low dose. Also, for example, if the expression level of NYAP2 of a subject is low and the sensitivity is predicted to be low, treatment may be performed by not administering BCV or by administering it at a high dose.

### (15) Method

An embodiment of the present invention provides a method for treating glioma, comprising the step of administering BCV, a pharmaceutically acceptable salt thereof, or a solvate thereof to a GPC6-low-expressing or NYAP2-high-expressing-glioma subject. By using this method, more appropriate BCV treatment can be performed by administering it to a subject having a GPC6-low-expressing or NYAP2-high-expressing, and BCV-highly sensitive glioma.

### (16) Method

An embodiment of the present invention provides a method for inhibiting the proliferation of glioma cells, comprising the step of contacting BCV, a pharmaceutically acceptable salt thereof, or a solvate thereof with glioma cells with a low expression level of GPC6 (GPC6-low-expressing glioma cells) or glioma cells with a high expression level of NYAP2 (NYAP2-high-expressing glioma cells). By using this method, a particularly high inhibitory effect on the proliferation of glioma cells can be obtained by contacting BCV with GPC6-low-expressing or NYAP2-high-expressing, and BCV-highly sensitive glioma cells.

### (17) Method

An embodiment of the present invention provides a method for predicting the sensitivity to BCV, a pharmaceutically acceptable salt thereof, or a solvate thereof in a subject with glioma, the method comprising the step of testing the expression level of GPC6 or NYAP2 in a sample derived from the subject with glioma. This method can be used to predict or identify a subject with high or low sensitivity to BCV based on the expression level of GPC6 or NYAP2 as an indicator. Therefore, by using this method, more appropriate BCV treatment can be performed. For example, if the expression level of GPC6 of a subject is low and the sensitivity is predicted to be high, treatment may be performed by administering BCV or by administering it at a low dose. For example, if the expression level of GPC6 of a subject is high and the sensitivity is predicted to be low, treatment may be performed by not administering BCV or by administering it at a high dose. For example, if the expression level of NYAP2 of a subject is high and the sensitivity is predicted to be high, treatment may be performed by administering BCV or by administering it at a low dose. For example, if the expression level of NYAP2 of a subject is low and the sensitivity is predicted to be low, treatment may be performed by not administering BCV or by administering it at a high dose. From another aspect, an embodiment of the present invention provides a method for predicting the sensitivity to BCV, a pharmaceutically acceptable salt thereof, or a solvate thereof in a subject with glioma, the method comprising the step of testing the expression level of GPC6 or NYAP2 in glioma cells derived from the subject with glioma.

### (18) Method

An embodiment of the present invention provides a method for determining a subject with glioma to whom BCV, a pharmaceutically acceptable salt thereof, or a solvate thereof is to be administered, the method comprising the step of testing the expression level of GPC6 or NYAP2 in a sample derived from the subject with glioma. By using this method, more appropriate BCV treatment can be performed. For example, if the expression level of GPC6 of a subject is low and the sensitivity is predicted to be high, the subject may be determined as a subject for administration of BCV or as a subject for administration at a low dose. For example, if the expression level of GPC6 of a subject is high and the sensitivity is predicted to be low, the subject may be determined as a subject for administration of BCV at a high dose. For example, if the expression level of NYAP2 of a subject is high and the sensitivity is predicted to be high, the subject may be determined as a subject for administration of BCV or as a subject for administration at a low dose. For example, if the expression level of NYAP2 of a subject is low and the sensitivity is predicted to be low, the subject may be determined as a subject for administration of BCV at a high dose. The subject to be administered may be a subject who should be administered. From another aspect, an embodiment of the present invention provides a method for selecting a subject with glioma suitable for treatment with BCV, a pharmaceutically acceptable salt thereof, or a solvate thereof, the method comprising the step of testing the expression level of GPC6 or NYAP2 in a sample derived from the subject with glioma.

### (19) Method

An embodiment of the present invention provides a method for diagnosing whether a subject is a subject having a glioma that is highly sensitive to BCV, a pharmaceutically acceptable salt thereof, or a solvate thereof, the method comprising the step of testing the expression level of GPC6 or NYAP2 in a sample derived from the subject with glioma. This method can be used to diagnose whether a subject has a glioma that is highly sensitive to BCV based on the expression level of GPC6 or NYAP2 as an indicator. Therefore, by using this method, more appropriate BCV treatment can be performed. For example, if a subject is diagnosed as having a glioma with a low expression level of GPC6 and high sensitivity, treatment may be performed by administering BCV or by administering it at a low dose. For example, if a subject is diagnosed as not having a glioma with a high expression level of GPC6 and high sensitivity, treatment may be performed by not administering BCV or by administering it at a high dose. For example, if a subject is diagnosed as having a glioma with a high expression level of NYAP2 and high sensitivity, treatment may be performed by administering BCV or by administering it at a low dose. For example, if a subject is diagnosed as not having a glioma with a low expression level of NYAP2 and high sensitivity, treatment may be performed by not administering BCV or by administering it at a high dose. This diagnosis may be a companion diagnosis for treatment with BCV, a pharmaceutically acceptable salt thereof, or a solvate thereof.

### (20) Method

An embodiment of the present invention provides a method for detecting the expression level of GPC6 or NYAP2 from glioma cells of a subject with a glioma sensitive to BCV, a pharmaceutically acceptable salt thereof, or a solvate thereof, the method comprising the steps of: (a) obtaining a sample from the subject (wherein the sample may be a glioma cell, a glioma cell lysate, or a solution containing a nucleic acid or protein contained in a glioma cell), or (b) contacting the sample with a GPC6 or NYAP2 detection reagent (e.g., including a nucleic acid or peptide described below), and then detecting the expression level of GPC6 or NYAP2 in the sample. According to this method, the expression level of GPC6 or NYAP2 from glioma cells of a subject with a BCV-sensitive glioma can be detected. For example, by detecting a low expression level of GPC6 or a high expression level of NYAP2 in GPC6-low-expressing or NYAP2-high-expressing glioma cells, treatment can be performed while anticipating that an effective therapeutic effect will be seen with BCV. For example, by detecting a low expression level of GPC6 or a high expression level of NYAP2 in GPC6-low-expressing or NYAP2-high-expressing glioma cells, it can be used as an indicator for setting the dose of BCV.

### (21) Method

An embodiment of the present invention provides a method for detecting an indicator for identifying a subject with glioma who has a high probability of being sensitive to BCV, a pharmaceutically acceptable salt thereof, or a solvate thereof, the method comprising the step of testing the expression level of GPC6 or NYAP2 in a sample derived from the subject with glioma. This method can be used to identify a subject with glioma who has a high probability of being sensitive to BCV based on the expression level of GPC6 or NYAP2 as an indicator. Therefore, by using this method, more appropriate BCV treatment can be performed.

### (22) Method

An embodiment of the present invention provides a method for detecting an indicator for identifying a subject with glioma who has a high probability of responding or reacting to BCV, a pharmaceutically acceptable salt thereof, or a solvate thereof, the method comprising the step of testing the expression level of GPC6 or NYAP2 in a sample derived from the subject with glioma. This method can be used to identify a subject with glioma who has a high probability of responding or reacting to BCV based on the expression level of GPC6 or NYAP2 as an indicator. Therefore, by using this method, more appropriate BCV treatment can be performed.

### (23) Method

An embodiment of the present invention provides a method for detecting a low expression level of GPC6 or a high expression level of NYAP2 from glioma cells of a subject to be administered with BCV, a pharmaceutically acceptable salt thereof, or a solvate thereof, or from glioma cells of a subject for whom such administration is being considered. This method includes, for example, the steps of: (a) obtaining a sample from the subject (wherein the sample may be a glioma cell, a glioma cell lysate, or a solution containing a nucleic acid or protein contained in a glioma cell), or (b) contacting the sample with a GPC6 or NYAP2 detection reagent (e.g., including a nucleic acid or peptide described below), and then detecting a low expression level of GPC6 or a high expression level of NYAP2 in the sample. According to this method, for example, by detecting a low expression level of GPC6 or a high expression level of NYAP2 in GPC6-low-expressing or NYAP2-high-expressing glioma cells, treatment can be performed while anticipating that an effective therapeutic effect will be seen with BCV.

### (24) Method

An embodiment of the present invention provides a method for predicting the efficacy of BCV, a pharmaceutically acceptable salt thereof, or a solvate thereof in a subject with glioma, the method comprising the step of testing the expression level of GPC6 or NYAP2 in a sample derived from the subject with glioma. This method can be used to identify a subject with high efficacy of BCV based on the expression level of GPC6 or NYAP2 as an indicator. Therefore, by using this method, more appropriate BCV treatment can be performed.

### (25) Method

An embodiment of the present invention provides a method for selecting a subject with glioma for whom a therapeutic effect with BCV, a pharmaceutically acceptable salt thereof, or a solvate thereof can be expected, the method comprising the step of testing the expression level of GPC6 or NYAP2 in a sample derived from the subject with glioma. This method can be used to select a subject for whom a therapeutic effect with BCV can be expected, based on the expression level of GPC6 or NYAP2 as an indicator. Therefore, by using this method, more appropriate BCV treatment can be performed.

### (26) Method

An embodiment of the present invention provides a method for obtaining an indicator for determining whether to perform a treatment method with BCV, a pharmaceutically acceptable salt thereof, or a solvate thereof, the method comprising the step of testing the expression level of GPC6 or NYAP2 in a sample derived from a subject with glioma. By using this method, more appropriate BCV treatment can be performed.

### (27) Method

An embodiment of the present invention provides a method for testing the expression level of GPC6 or the high expression level of NYAP2 in a sample from a subject who has or is suspected of having glioma. This method may include, for example, a step of measuring the expression level of GPC6 or NYAP2 in a sample from the subject, or a step of identifying a subject who provided a GPC6-low-expressing sample or a NYAP2-high-expressing sample as a subject for administration of BCV, a pharmaceutically acceptable salt thereof, or a solvate thereof. By using this method to identify a subject having a GPC6-low-expressing or NYAP2-high-expressing glioma, more appropriate BCV treatment can be performed.

### (28) Method

An embodiment of the present invention provides a method for selecting a treatment method or an active ingredient for use in treatment, the method comprising the step of using the expression level of GPC6 or NYAP2 as an indicator. This method may include, for example, the step of testing the expression level of GPC6 or NYAP2 in a sample derived from a subject with glioma. This method may, for example, select to use BCV, a pharmaceutically acceptable salt thereof, or a solvate thereof for treatment if the subject's GPC6 is predicted to be low-expressing and highly sensitive to BCV. This method may, for example, select to use BCV, a pharmaceutically acceptable salt thereof, or a solvate thereof for treatment if the subject's NYAP2 is predicted to be high-expressing and highly sensitive to BCV. This method may, for example, adjust or select the dosage of BCV, a pharmaceutically acceptable salt thereof, or a solvate thereof based on the expression level of GPC6 or NYAP2 as an indicator. The treatment method may include the step of administering brincidofovir, a pharmaceutically acceptable salt thereof, or a solvate thereof to a subject. This method may be a companion diagnostic method for selecting a treatment method or an active ingredient for use in treatment.

### (29) Method

An embodiment of the present invention provides a method for predicting BCV sensitivity in tumor cell proliferation, the method comprising the step of testing the expression level of GPC6 or NYAP2 in a sample derived from a subject with glioma. By using this method, tumor cell proliferation can be more effectively inhibited.

### (30) Method

An embodiment of the present invention provides a method for predicting drug sensitivity or a method for determining a subject for drug administration, the method comprising the step of testing the expression level of GPC6 or NYAP2 in a sample derived from a subject with glioma. By using this method, glioma can be treated more effectively.

### (31) Method

According to an embodiment of the present invention, in the methods described in (14) to (30) above, one or more of RFTN2, JAKMIP2, SORBS2, GALNT13, RFTREG1, STXBP6, or DCC (hereinafter referred to as RFTN2 and the like) may be used as an indicator or a testing target instead of or in addition to GPC6. In this case, the embodiments for RFTN2 and the like may adopt the same embodiments as for GPC6 (e.g., (1) to (30) above) (in this case, GPC6 may be read as RFTN2 and the like). According to an embodiment of the present invention, in the methods described in (1) to (30) above, one or more of CCDC3, OLR1, or EEF1A2 (hereinafter referred to as CCDC3 and the like) may be used as an indicator or a testing target instead of or in addition to NYAP2. In this case, the embodiments for CCDC3 and the like may adopt the same embodiments as for NYAP2 (e.g., (1) to (30) above) (in this case, NYAP2 may be read as CCDC3 and the like).

### (32) Composition

An embodiment of the present invention provides a pharmaceutical composition for treatment of glioma, comprising BCV, a pharmaceutically acceptable salt thereof, or a solvate thereof. This composition can be used to treat glioma through a novel therapeutic approach. Using this composition, for example, a treatment with an excellent balance between therapeutic effect and safety can be performed. Using this composition, for example, glioma can be treated with a dose suitable for clinical application (e.g., a dose with excellent efficacy and safety) or a low dose of the agent. Using this composition, for example, it is possible to treat glioblastoma, which is a highly malignant type of glioma and is generally difficult to treat. Using this composition, for example, the life of a subject with glioma can be prolonged with a dose suitable for clinical application or a low dose of the agent. From another aspect, an embodiment of the present invention provides a pharmaceutical composition comprising BCV, a pharmaceutically acceptable salt thereof, or a solvate thereof for use in the treatment of glioma.

### (33) Composition

An embodiment of the present invention provides a pharmaceutical composition for treatment of glioma, comprising BCV, a pharmaceutically acceptable salt thereof or a solvate thereof, or a chemotherapeutic agent, for use in a combination treatment with BCV, a pharmaceutically acceptable salt thereof, or a solvate thereof, and a chemotherapeutic agent. This composition can be used to treat glioma through a novel therapeutic approach. Using this composition, for example, a treatment with an excellent balance between therapeutic effect and safety can be performed. Using this composition, for example, glioma can be treated with a dose suitable for clinical application (e.g., a dose with excellent efficacy and safety) or a low dose of the agent. Using this composition, for example, the life of a subject with glioma can be prolonged with a dose suitable for clinical application or a low dose of the agent. Using this composition, for example, a synergistic or additive effect can be obtained by the combination of BCV and the chemotherapeutic agent. The chemotherapeutic agent is preferably TMZ, a pharmaceutically acceptable salt thereof, or a solvate thereof, from the viewpoint that a significant therapeutic effect is exerted in combination with a lower dose of BCV.

### (34) Composition

An embodiment of the present invention provides a pharmaceutical composition for treatment of glioma, comprising BCV, a pharmaceutically acceptable salt thereof, or a solvate thereof, for administration to a subject, wherein the subject is a subject to whom a chemotherapeutic agent is administered prior to, concurrently with, or subsequent to the administration of BCV, a pharmaceutically acceptable salt thereof, or a solvate thereof. Using this composition, the same effect as in the composition of (33) above can be obtained. Concurrent administration includes administration within the same time period and any administration performed substantially concurrently, taking usual treatment procedures into account. Concurrent administration includes administration of BCV and a chemotherapeutic agent as a combination preparation. From another aspect, an embodiment of the present invention provides a pharmaceutical composition for treatment of glioma, comprising a chemotherapeutic agent, for administration to a subject, wherein the subject is a subject to whom BCV, a pharmaceutically acceptable salt thereof, or a solvate thereof is administered prior to, concurrently with, or subsequent to the administration of the chemotherapeutic agent. From another aspect, an embodiment of the present invention provides a pharmaceutical composition for treatment of glioma, comprising BCV, a pharmaceutically acceptable salt thereof, or a solvate thereof, for administration to a subject to whom a chemotherapeutic agent has been administered. From another aspect, an embodiment of the present invention provides a pharmaceutical composition for treatment of glioma, comprising a chemotherapeutic agent, for administration to a subject to whom BCV, a pharmaceutically acceptable salt thereof, or a solvate thereof has been administered. From another aspect, an embodiment of the present invention provides a pharmaceutical composition for treatment of glioma, comprising BCV, a pharmaceutically acceptable salt thereof, or a solvate thereof, and a chemotherapeutic agent.

### (35) Composition

An embodiment of the present invention provides a pharmaceutical composition or a kit, comprising BCV, a pharmaceutically acceptable salt thereof, or a solvate thereof, and a chemotherapeutic agent. This composition or kit can be used to treat glioma through a novel therapeutic approach. Using this composition or kit, for example, a treatment with an excellent balance between therapeutic effect and safety can be performed. Using this composition or kit, for example, glioma can be treated with a dose suitable for clinical application (e.g., a dose with excellent efficacy and safety) or a low dose of the agent. Using this composition or kit, for example, the life of a subject with glioma can be prolonged with a dose suitable for clinical application or a low dose of the agent. The chemotherapeutic agent is preferably TMZ, a pharmaceutically acceptable salt thereof, or a solvate thereof, from the viewpoint that a significant therapeutic effect is exerted in combination with a lower dose of BCV.

### (36) Composition

An embodiment of the present invention provides a pharmaceutical composition for treatment of glioma, comprising BCV, a pharmaceutically acceptable salt thereof or a solvate thereof, for use in a combination treatment with BCV, a pharmaceutically acceptable salt thereof, or a solvate thereof, and radiation therapy. This composition can be used to treat glioma through a novel therapeutic approach. Using this composition, for example, a treatment with an excellent balance between therapeutic effect and safety can be performed. Using this composition, for example, glioma can be treated with a dose suitable for clinical application (e.g., a dose with excellent efficacy and safety) or a low dose of the agent. Using this composition, for example, the life of a subject with glioma can be prolonged with a dose suitable for clinical application or a low dose of the agent. Using this composition, for example, a synergistic or additive effect of BCV and radiation therapy can be obtained.

### (37) Composition

An embodiment of the present invention provides a pharmaceutical composition for treatment of glioma, comprising BCV, a pharmaceutically acceptable salt thereof or a solvate thereof, or a chemotherapeutic agent, for use in a combination treatment with (a) BCV, a pharmaceutically acceptable salt thereof, or a solvate thereof, (b) a chemotherapeutic agent, and (c) radiation therapy. This composition can be used to treat glioma through a novel therapeutic approach. Using this composition, for example, a treatment with an excellent balance between therapeutic effect and safety can be performed. Using this composition, for example, glioma can be treated with a dose suitable for clinical application (e.g., a dose with excellent efficacy and safety) or a low dose of the agent. Using this composition, for example, the life of a subject with glioma can be prolonged with a dose suitable for clinical application or a low dose of the agent. Using this composition, for example, a synergistic or additive effect of BCV, the chemotherapeutic agent, and radiation therapy can be obtained.

### (38) Composition

An embodiment of the present invention provides a pharmaceutical composition for treatment of glioma, comprising BCV, a pharmaceutically acceptable salt thereof, or a solvate thereof, wherein the subject of the treatment is a subject identified based on the expression level of GPC6 or NYAP2 as an indicator. By using this pharmaceutical composition, sensitivity can be predicted based on the expression level of GPC6 or NYAP2 as an indicator, and more appropriate BCV treatment can be performed.

### (39) Composition

An embodiment of the present invention provides a composition for use in predicting sensitivity to BCV, a pharmaceutically acceptable salt thereof, or a solvate thereof in a subject with glioma, the composition comprising a GPC6 or NYAP2 expression level testing reagent. This composition can be used to predict or identify a subject with high or low sensitivity to BCV based on the expression level of GPC6 or NYAP2 as an indicator. Therefore, by using this composition, more appropriate BCV treatment can be performed.

### (40) Composition

An embodiment of the present invention provides a composition for use in determining a subject with glioma to whom BCV, a pharmaceutically acceptable salt thereof, or a solvate thereof is to be administered, the composition comprising a GPC6 or NYAP2 expression level testing reagent. By using this composition, more appropriate BCV treatment can be performed.

### (41) Composition

An embodiment of the present invention provides a composition for use in diagnosing whether a subject is a subject having a glioma that is highly sensitive to brincidofovir, a pharmaceutically acceptable salt thereof, or a solvate thereof, the composition comprising a GPC6 or NYAP2 expression level testing reagent. This composition can be used to diagnose whether a subject has a glioma that is highly sensitive to BCV based on the expression level of GPC6 or NYAP2 as an indicator. Therefore, by using this composition, more appropriate BCV treatment can be performed.

### (42) Composition

An embodiment of the present invention provides a composition for use in detecting an indicator for identifying a subject with glioma who has a high probability of being sensitive to BCV, a pharmaceutically acceptable salt thereof, or a solvate thereof, the composition comprising a GPC6 or NYAP2 expression level testing reagent. From another aspect, an embodiment of the present invention provides a composition for use in detecting an indicator for identifying a subject with glioma who has a high probability of responding or reacting to BCV, a pharmaceutically acceptable salt thereof, or a solvate thereof, the composition comprising a GPC6 or NYAP2 expression level testing reagent. From another aspect, an embodiment of the present invention provides a composition for use in detecting the expression level of GPC6 or NYAP2 in a sample from a subject who has or is suspected of having glioma, the composition comprising a GPC6 or NYAP2 expression level testing reagent. By using any of these methods, more appropriate BCV treatment can be performed.

### (43) Composition

An embodiment of the present invention provides a composition for use in the methods described in (1) to (31) above, comprising a reagent for testing the expression level of a protein or gene such as GPC6, NYAP2, RFTN2 and the like, or CCDC3 and the like.

### (44) Composition

An embodiment of the present invention provides a composition for use in the methods described in (1) to (31) above, comprising BCV, a pharmaceutically acceptable salt thereof or a solvate thereof, or a chemotherapeutic agent.

### (45) Kit

An embodiment of the present invention provides a kit for use in the methods described in (1) to (31) above, comprising a reagent for testing the expression level of a protein or gene such as GPC6, NYAP2, RFTN2 and the like, or CCDC3 and the like. The kit may include, for example, instructions for use, a buffer, a container, or packaging.

### (46) Kit

An embodiment of the present invention provides a kit for use in the methods described in (1) to (31) above, comprising BCV, a pharmaceutically acceptable salt thereof or a solvate thereof, or a chemotherapeutic agent. The kit includes a kit for treating glioma, for inhibiting the proliferation of glioma cells, or for prolonging the life of a patient with glioma. The kit may include, for example, instructions for use, a buffer, a container, or packaging.

### (47) Use

An embodiment of the present invention provides a use of a protein or gene such as GPC6, NYAP2, RFTN2 and the like, or CCDC3 and the like as a biomarker for use in the methods described in (1) to (31) above.

### (48) Use

An embodiment of the present invention provides a use of BCV, a pharmaceutically acceptable salt thereof, or a solvate thereof, or a chemotherapeutic agent for the manufacture of the compositions described in (32) to (44) above.

In embodiments (e.g., (1) to (48) above) of the present invention, brincidofovir (BCV) includes a compound having a structure represented by the following formula. BCV can also be represented by the IUPAC name [(2S)-1-(4-amino-2-oxopyrimidin-1-yl)-3-hydroxypropan-2-yl]oxymethyl-(3-hexadecoxypropoxy)phosphinic acid. BCV includes the compound represented by CAS Registry Number 444805-28-1. Herein, BCV is an abbreviation for brincidofovir, and they have the same meaning. In the Examples described below, it was shown that as a result of administering BCV to an animal with glioma, a treatment with an excellent balance between therapeutic effect and suppression of systemic toxicity can be performed. Although several research reports on BCV have been published in the past, there are no reports demonstrating a therapeutic effect in animals with glioma. In addition, past research reports have not shown that a dose of BCV suitable for treatment or a low dose of BCV is effective when targeting glioma. BCV is known to have an antiviral effect, but its mechanism of action is the inhibition of viral replication by inhibiting virus-derived DNA polymerase, which is considered to be different from the mechanism of action against solid cancers such as glioma. For example, Table 4 of JP 2013-501056 A describes the in vitro IC₅₀ values of BCV for renal cancer cell lines and the like, which are very large compared to the values reported for antiviral activity. This suggests that when the target is a renal cancer cell line, a larger amount of BCV is required than when the target is a virus.

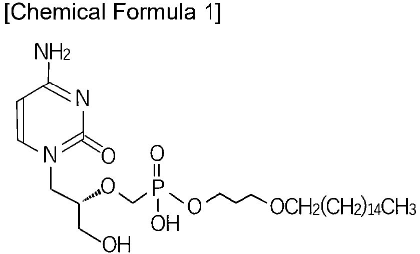

In embodiments (e.g., (1) to (48) above) of the present invention, glioma is a type of malignant brain tumor. Glioma includes, for example, glioblastoma. Glioblastoma can be classified as the most malignant tumor among gliomas.

In embodiments (e.g., (1) to (48) above) of the present invention, the glioma may be an MGMT gene promoter-unmethylated glioma (e.g., glioblastoma). In this case, an excellent therapeutic effect by BCV can be obtained for a glioma that has resistance to TMZ. An MGMT gene promoter-unmethylated glioma includes, for example, a glioblastoma in which the promoter region of the MGMT gene is not methylated. The presence or absence of unmethylation may be tested, for example, using real-time methylation-specific PCR (see, e.g., Yoshioka et al., Oncotarget. 2018 Jun 12;9(45):27728-27735), a pyrosequencing method (see, e.g., Berghoff et al., CNS Oncol. 2015;4(1):47-52.), or a commercially available testing kit (e.g., CpG WIZ(R) MGMT - Methylation specific PCR assay (Merck)). MGMT can also be denoted as O6-methylguanine-DNA methyltransferase. The primary accession number for MGMT described in UniProt is, for example, P16455.

In embodiments (e.g., (1) to (48) above) of the present invention, the glioma may be a CMV (cytomegalovirus)-positive glioma. In this case, a particularly excellent therapeutic effect by BCV can be obtained. A CMV-positive glioma includes, for example, a glioma caused by CMV infection or a glioma that has become malignant due to CMV infection.

In embodiments (e.g., (1) to (48) above) of the present invention, the glioma may be a GPC6-low-expressing or NYAP2-high-expressing glioma. In this case, an improvement in the probability of a therapeutic effect appearing or an improvement in the therapeutic effect can be expected, compared to when BCV treatment is performed on an unspecified patient.

The methods of the embodiments (e.g., (1) to (31) above) of the present invention may include, for example, the step of identifying or selecting a subject having a glioma. The identifying or selecting step may be performed using a glioma biomarker in a body fluid (e.g., blood) of a test subject as an indicator, or by diagnostic imaging (CT, etc.) or brain tissue findings. The identifying or selecting step may be performed based on an increase in the amount of a biomarker in the test subject compared to the amount of the glioma biomarker in a comparison subject (e.g., a healthy person) as an indicator. The identifying or selecting step may be performed, for example, using the diagnostic method described in Silantyev et al., Cells. 2019 Aug 9;8(8):863. The methods of the embodiments (e.g., (1) to (31) above) of the present invention may include, for example, the step of treating the glioma of the subject identified or selected by the above step.

In embodiments (e.g., (1) to (48) above) of the present invention, the subject (including a patient) may be, for example, a subject diagnosed as having developed glioma, or a subject in need of treatment for glioma. The subject may be, for example, a subject in whom a glioma biomarker has increased compared to a comparison subject (e.g., a healthy person), or a subject in whom glioma has been observed by diagnostic imaging or brain tissue findings. The subject may be, for example, a subject who has experienced glioma. The subject includes a human or a non-human mammal (e.g., one or more species such as a mouse, a guinea pig, a hamster, a rat, a mouse, a rabbit, a pig, a sheep, a goat, a cow, a horse, a cat, a dog, a marmoset, a monkey, or a chimpanzee). The subject includes a human adult patient (e.g., a human patient with a body weight of 50 or 60 kg or more). The subject may be, for example, a subject having an MGMT gene promoter-unmethylated glioma or a CMV-positive subject. The subject may be, for example, an MGMT-high-expressing or -positive subject. The subject may be, for example, a GPC6-low-expressing or NYAP2-high-expressing subject. The subject may be, for example, a subject who is positive or negative, or wild-type or mutant-type, for EGFR, PTEN, p53, or p16.

The methods of the embodiments (e.g., (1) to (31) above) of the present invention may include, for example, a step of identifying a subject having a glioma; a step of identifying a subject who is positive for a glioma marker; a step of administering a therapeutically effective amount of BCV or a chemotherapeutic agent to a subject; a step of suppressing the proliferation of glioma cells in a subject; or a step of reducing a glioma marker in a subject. Also, the methods of the embodiments (e.g., (1) to (31) above) of the present invention optionally may or may not include, for example: a step of identifying a subject having an adenovirus (AdV), BK virus (BKV), variola virus (VaV), or Epstein-Barr virus (EBV) infection; a step of identifying an AdV-, BKV-, VaV-, or EBV-seropositive subject; a step of identifying a subject in need of prevention or treatment of AdV, BKV, VaV, or EBV infection; a step of identifying a subject after allogeneic transplantation (e.g., hematopoietic cell transplantation); a step of identifying a subject in need of an immunosuppressive agent; a step of identifying a subject in an immunosuppressed state; a method for treating a virus-induced tumor in a subject in an immunosuppressed state; or a method for preventing or treating a viral (e.g., AdV, BKV, VaV, or EBV) infection.

The methods of the embodiments (e.g., (1) to (31) above) of the present invention may include, for example, (i) a step of identifying a subject having an MGMT gene promoter-unmethylated glioma, (ii) a step of detecting MGMT gene promoter unmethylation of the subject's glioma, (iii) a step of detecting the presence or absence of MGMT gene promoter unmethylation of the subject's glioma, (iv) a step of collecting a specimen (e.g., glioma cells) from the subject, (v) a step of detecting MGMT gene promoter unmethylation in the collected specimen, (vi) a step of identifying a subject who provided a specimen in which MGMT gene promoter unmethylation was detected, (vii) a step of extracting DNA (e.g., genomic DNA) from glioma cells, or (viii) a step of detecting the presence or absence of MGMT gene promoter unmethylation in the extracted DNA. Adopting one or more of these steps is useful for providing treatment to a population in which the treatment is highly effective. When two or more are adopted, their order is arbitrary and can be determined according to the desired treatment method. These steps may be performed prior to or subsequent to the step of administering BCV, a pharmaceutically acceptable salt thereof, or a solvate thereof, or a chemotherapeutic agent to a subject. The detection may be a test.

The methods of the embodiments (e.g., (1) to (31) above) of the present invention may include, for example, (i) a step of identifying a CMV-positive subject, (ii) a step of identifying a subject having a CMV-positive glioma, (iii) a step of detecting CMV in a subject, (iv) a step of detecting the presence or absence of CMV positivity in a subject, (v) a step of collecting a specimen from a subject (e.g., brain tissue, or a body fluid (e.g., a blood specimen (e.g., plasma, serum, or whole blood))), (vi) a step of detecting CMV in the collected specimen, or (vii) a step of identifying a subject who provided a specimen in which CMV was detected. Adopting one or more of these steps is useful for providing treatment to a population in which the treatment is highly effective. When two or more are adopted, their order is arbitrary and can be determined according to the desired treatment method. These steps may be performed prior to or subsequent to the step of administering BCV, a pharmaceutically acceptable salt thereof, or a solvate thereof, or a chemotherapeutic agent to a subject. The detection may be based on the amount of CMV nucleic acid (e.g., gene amount or mRNA amount) or the amount of protein as an indicator. The detection may be a test.

In embodiments (e.g., (1) to (48) above) of the present invention, positivity includes that gene expression is positive. Positivity may include that gene expression is increased compared to that in a healthy person or a person previously confirmed to be negative. In embodiments (e.g., (1) to (48) above) of the present invention, the detection of CMV positivity/negativity of a subject or a subject's glioma can be performed, for example, by collecting a specimen from the subject and measuring the gene expression of CMV in the specimen. Gene expression can be measured, for example, by RT-PCR, DNA chip, or immunostaining. In this case, a specimen collected from the subject may be compared with a specimen collected from a healthy person or a person previously confirmed to be negative, and when the gene expression of CMV is significantly enhanced in the specimen collected from the subject, it may be determined to be CMV-positive. The enhancement of gene expression includes, for example, an increase of 1.5, 2, 3, 4, 5, 10, 20, or 50 times, or within a range between any two of these values, relative to a comparison subject. The detection of CMV positivity/negativity may be performed, for example, in vitro, ex vivo, or in vivo.

The methods of the embodiments (e.g., (1) to (31) above) of the present invention may be performed, for example, in a combination treatment of administration of BCV, a pharmaceutically acceptable salt thereof, or a solvate thereof, and radiation therapy, or in a combination treatment of (a) administration of BCV, a pharmaceutically acceptable salt thereof, or a solvate thereof, (b) administration of a chemotherapeutic agent, and (c) radiation therapy.

In embodiments (e.g., (1) to (48) above) of the present invention, combination includes, when two or more treatments (e.g., administrations) are present, performing another treatment prior to, concurrently with, or subsequent to one treatment as a reference.

In embodiments (e.g., (1) to (48) above) of the present invention, treatment includes being able to exert an action that brings a beneficial result to a patient with respect to the patient's disease (e.g., glioma (e.g., glioblastoma)) or one or more symptoms associated with the disease (e.g., improvement, reduction, alleviation, cure, remission, or suppression (e.g., suppression of onset (e.g., prevention), suppression of progression, or suppression of recurrence)). The treatment may be performed by administering a therapeutically effective amount of BCV, a pharmaceutically acceptable salt thereof, or a solvate thereof to a subject. In embodiments (e.g., (1) to (48) above) of the present invention, the pharmaceutical composition may be manufactured by any method known in the technical field of pharmaceutics, for example, by mixing the active ingredient with one or more pharmaceutically acceptable carriers. Also, the pharmaceutical composition is not limited in its form of use as long as it is used for treatment, and may be the active ingredient alone or a mixture of the active ingredient and any components. Also, the shape of the carrier is not particularly limited, and may be, for example, a solid or a liquid (e.g., a buffer). The content of the carrier may be, for example, a pharmaceutically effective amount. The effective amount may be, for example, an amount sufficient for the pharmaceutical stability or delivery of the active ingredient. For example, a buffer is effective for stabilizing the active ingredient in a vial. The pharmaceutical composition may also include a stabilizer, a buffer, or a pH adjuster. The dosage, administration interval, administration method, and administration route are not particularly limited and can be appropriately selected according to the patient's age, body weight, symptoms, target organ, and the like. The pharmaceutical composition preferably contains a therapeutically effective amount, or an effective amount of the active ingredient that exerts a desired action. In one embodiment of the present invention, a therapeutically effective amount includes the amount necessary for a clinically observed improvement in symptoms in a patient. In one embodiment of the present invention, pharmaceutically acceptable includes a state suitable for use within the scope of sound medical judgment and commensurate with a reasonable benefit/risk ratio. Ingredients other than BCV in the pharmaceutical composition are not particularly limited as long as the effects of the present invention are not impaired, and can be appropriately selected according to the purpose. The therapeutic effect on glioma may be judged to be present if the patient's glioma cells are significantly reduced after administration of the therapeutic agent. The therapeutic effect may be based on the improvement of the patient's brain tissue findings as an indicator.

In embodiments (e.g., (1) to (48) above) of the present invention, the administration route of BCV, a pharmaceutically acceptable salt thereof, or a solvate thereof (or a pharmaceutical composition containing them) to a subject (e.g., a human subject) is preferably one that is effective for treatment, and may be, for example, intravenous, oral, subcutaneous, intramuscular, or intraperitoneal. The dosage form is preferably one that is effective for treatment, and may be, for example, a solid formulation (e.g., a tablet), a liquid formulation (e.g., an oral solution), or an injection (e.g., an intravenous injection).

In embodiments (e.g., (1) to (48) above) of the present invention, the dosage, administration interval, and administration method of BCV, a pharmaceutically acceptable salt thereof, or a solvate thereof (or a pharmaceutical composition containing them) to a subject (e.g., a human subject) can be appropriately selected according to the patient's age, body weight, symptoms, target organ, and the like. This dosage may be, for example, 1 to 300 mg per administration. Herein, unless otherwise specified, mg of a dosage indicates the amount administered per individual (body) of the administration subject. More specifically, the dosage includes, for example, 5-100, 10-90, 10-80, 10-60, 10-50, 15-70, 15-50, 20-70, 20-50, 30-70, 30-60, 30-50, 35-45, or 38-42 mg. Also, the dosage of BCV during combination treatment with a chemotherapeutic agent (e.g., TMZ) includes, for example, 5-100, 10-90, 10-80, 10-60, 10-50, 15-70, 15-50, 15-30, 15-25, 18-22, 20-70, 20-50, 30-70, 30-60, 30-50, or 35-45 mg. The above dosage may be, for example, 1, 5, 10, 15, 20, 25, 30, 35, 40, 45, 50, 55, 60, 65, 70, 75, 80, 85, 90, 95, 100, or 300 mg, and may be greater than or equal to any of these values, or within a range between any two of these values. The dosages shown here may each be a dosage per administration or per day. The administration interval may be, for example, once or twice every 1 to 28 days or every 1 to 4 weeks. The administration period may be, for example, 2 to 10 or 4 to 6 weeks. For example, 20-40 mg of BCV may be administered once or twice a week for 6 weeks to a human patient by intravenous injection. From another aspect, the dosage may be, for example, 0.02 to 6 mg/kg per administration. More specifically, the dosage includes 0.1-2, 0.2-1.8, 0.2-1.6, 0.2-1.2, 0.2-1, 0.3-1.4, 0.3-1, 0.3-0.6, 0.3-0.5, 0.36-0.44, 0.4-1.4, 0.4-1, 0.6-1.4, 0.6-1.2, 0.6-1, or 0.7-0.9 mg/kg. Also, the dosage of BCV during combination treatment with a chemotherapeutic agent (e.g., TMZ) includes, for example, 0.1-2, 0.2-1.8, 0.2-1.6, 0.2-1.2, 0.2-1, 0.3-1.4, 0.3-1, 0.3-0.8, 0.3-0.6, 0.6-1.4, 0.6-1.2, 0.6-1, or 0.7-0.9 mg/kg. The above dosage may be, for example, 0.02, 0.5, 0.1, 0.2, 0.3, 0.36, 0.4, 0.44, 0.5, 0.6, 0.7, 0.8, 0.9, 1, 1.2, 1.3, 1.4, 1.5, 1.6, 1.8, 2, 3, 4, 5, or 6 mg, and may be greater than or equal to any of these values, or within a range between any two of these values. For example, 0.4-0.8 mg/kg of BCV may be administered once or twice a week for 6 weeks to a human patient by intravenous injection or oral administration.

In embodiments (e.g., (1) to (48) above) of the present invention, the dosage, administration interval, and administration method of a chemotherapeutic agent (e.g., TMZ, a pharmaceutically acceptable salt thereof, or a solvate thereof) or a pharmaceutical composition containing it to a subject (e.g., a human subject) during combination treatment can be appropriately selected according to the patient's age, body weight, symptoms, target organ, and the like. This dosage may be, for example, 1 to 500 mg/m² (body surface area) per administration. More specifically, the dosage includes, for example, 1-400, 5-200, 5-75, 5-30, 5-400, 30-300, 50-250, 50-200, 70-200, 70-170, or 130-170 mg/m². The above dosage may be, for example, 1, 5, 10, 20, 30, 50, 70, 75, 100, 130, 150, 170, 200, 300, 400, or 500 mg/m², and may be greater than or equal to any of these values, or within a range between any two of these values. The dosages shown here may each be a dosage per administration or per day. The administration interval may be, for example, once or twice every 1 to 28 days or every 1 to 4 weeks. The administration period may be, for example, 1 to 5 days or 1 to 6 weeks. For example, 75-200 mg/m² of a chemotherapeutic agent may be administered once a day for 5 consecutive days or for 6 consecutive weeks to a human patient by intravenous injection or oral administration. During combination treatment with a chemotherapeutic agent and BCV, the dosage, administration interval, and administration method of the chemotherapeutic agent shown here can be adopted in combination with the administration interval and administration method of BCV described above, respectively. (For example, BCV may adopt a dose in the range shown above within 1-300 mg, and the chemotherapeutic agent may adopt a dose in the range shown above within 1-500 mg/m² (body surface area). For example, 15-30 mg of BCV may be administered once or twice a week (e.g., for 6 weeks) to a human patient by intravenous injection, and 75-200 mg/m² of a chemotherapeutic agent may be administered once a day (e.g., for 5 consecutive days or for 6 consecutive weeks) to a human patient by intravenous injection or oral administration.) From another aspect, the dosage may be, for example, 1 to 500 mg per administration. More specifically, the dosage includes, for example, 1-400, 5-200, 5-75, 5-30, 5-400, 50-300, 50-250, 50-150, 100-150, 100-300, or 250-300 mg. The above dosage may be, for example, 1, 5, 10, 20, 30, 50, 75, 100, 135, 150, 200, 300, 400, or 500 mg, and may be greater than or equal to any of these values, or within a range between any two of these values. The dosage of 2 mg/kg TMZ to mice described in the Examples below can be converted to 9.6 mg/body for humans. Since this dosage is lower than the typical dosage of TMZ monotherapy, it is suggested that the combination of BCV and TMZ is a treatment method with excellent safety.

In embodiments (e.g., (1) to (48) above) of the present invention, the chemotherapeutic agent is not particularly limited and includes, for example, an anticancer agent. The anticancer agent includes, for example, a microtubule inhibitor, a DNA synthesis inhibitor, a growth factor inhibitor, a tyrosine kinase inhibitor, a cytotoxic substance, an immune checkpoint inhibitor, or other anticancer agents. The microtubule inhibitor includes, for example, a vinca alkaloid drug or a taxane drug. The vinca alkaloid drug includes, for example, vincristine, vinblastine, vindesine, vinorelbine, or eribulin. The taxane drug includes, for example, paclitaxel or docetaxel. The DNA synthesis inhibitor includes, for example, an antimetabolite, a topoisomerase inhibitor, a platinum formulation, an antitumor antibiotic, or an alkylating agent. The antimetabolite includes, for example, pemetrexed, 5-fluorouracil, S-1, gemcitabine, or capecitabine. The topoisomerase inhibitor includes, for example, irinotecan, nogitecan, etoposide, or sobuzoxane. The platinum formulation includes, for example, cisplatin, oxaliplatin, nedaplatin, or carboplatin. The antitumor antibiotic includes, for example, an anthracycline drug (e.g., doxorubicin, liposomal doxorubicin, daunorubicin, epirubicin, idarubicin, aclarubicin, amrubicin, mitoxantrone, or pirarubicin), mitomycin C, actinomycin D, bleomycin, peplomycin, or zinostatin stimalamer. The alkylating agent includes, for example, TMZ, a nitrosourea compound (e.g., lomustine, carmustine, etc.), bendamustine, cyclophosphamide, dacarbazine, or ifosfamide. The growth factor inhibitor includes, for example, an inhibitor of EGF, VEGF, FGF, or IGF. The growth factor inhibitor includes, for example, bevacizumab, cetuximab, panitumumab. The tyrosine kinase inhibitor includes, for example, gefitinib or erlotinib. The cytotoxic substance includes, for example, saporin, emtansine, deruxtecan, or vedotin. The immune checkpoint inhibitor includes, for example, an agent that binds to an immune checkpoint molecule or its ligand to inhibit the transmission of an immunosuppressive signal, thereby releasing the suppression of T-cell activation by the immune checkpoint molecule. The immune checkpoint inhibitor includes, for example, an anti-CTLA-4 antibody (e.g., ipilimumab), an anti-PD-1 antibody (e.g., nivolumab or pembrolizumab), or an anti-PD-L1 antibody (e.g., atezolizumab or avelumab). Other anticancer agents include, for example, L-asparaginase. The form of the chemotherapeutic agent includes, for example, a low molecular weight compound or a high molecular weight compound. The chemotherapeutic agent includes a salt (including the salts shown below) of any one or more of the compounds shown here.

In embodiments (e.g., (1) to (48) above) of the present invention, the salt is not particularly limited and includes, for example, an inorganic salt or an organic salt (see, e.g., Bharate et al., Drug Discov Today. 2021 Feb;26(2):384-398. or Berge et al., J Pharm Sci. 1977 Jan;66(1):1-19.). The salt includes, for example, a metal salt, an ammonium salt, a salt with an organic base, a salt with an inorganic acid, a salt with an organic acid, a salt with a basic or acidic amino acid, and the like. The metal salt includes, for example, an alkali metal salt (sodium salt, potassium salt, etc.), an alkaline earth metal salt (calcium salt, magnesium salt, barium salt, etc.), an aluminum salt, and the like. The salt with an organic base includes, for example, a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, and the like. The salt with an inorganic acid includes, for example, a salt with hydrochloric acid, hydrobromic acid, nitric acid, sulfuric acid, phosphoric acid, and the like. The salt with an organic acid includes, for example, a salt with formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, p-toluenesulfonic acid, and the like. The salt with a basic amino acid includes, for example, a salt with arginine, lysine, ornithine, and the like. The salt with an acidic amino acid includes, for example, a salt with aspartic acid, glutamic acid, and the like. The salt includes a pharmaceutically acceptable salt. In one embodiment of the present invention, pharmaceutically acceptable includes a form that has a reasonable benefit for pharmaceutical use. In one embodiment of the present invention, one form of a compound or a salt thereof includes a solvate form thereof. In embodiments (e.g., (1) to (48) above) of the present invention, a solvate includes a form of a compound formed by a solute and a solvent (see, e.g., Healy et al., Adv Drug Deliv Rev. 2017 Aug 1;117:25-46.). The solvate is not particularly limited, but includes, for example, a hydrate (e.g., a monohydrate, a dihydrate, a trihydrate, etc.) or an organic solvate (e.g., a solvate with an alcohol (methanol, ethanol, propanol, etc.), acetone, dimethylformamide, or ethyl acetate, etc.). The solvent includes a solvent that can substantially maintain the physiological activity of the solute after forming a solvate. The solvate includes a pharmaceutically acceptable solvate.

In embodiments (e.g., (1) to (48) above) of the present invention, radiation therapy includes, for example, treatment by irradiating a portion containing a tumor with radiation. The number of times, interval, duration, and absorbed dose of radiation therapy can be appropriately selected according to the patient's age, body weight, symptoms, target organ, and the like. For radiation therapy, for example, 1 to 2 Gray or 1.5 to 2 Gray may be irradiated once or twice a day.

In embodiments (e.g., (1) to (48) above) of the present invention, GPC6 includes the protein denoted as glypican-6. Details such as the amino acid sequence of GPC6 can be confirmed from websites such as UniProt or NCBI. The primary accession number for GPC6 in UniProt is Q9Y625, and the Gene ID in NCBI is 10082. In the Examples described below, GPC6 corresponding to the above Q9Y625 was detected. GPC6 may be, for example, GPC6 derived from a human, monkey, mouse, rat, rabbit, dog, or cat.

In embodiments (e.g., (1) to (48) above) of the present invention, NYAP2 includes the protein denoted as Neuronal Tyrosine-Phosphorylated Phosphoinositide-3-Kinase Adaptor 2. Details such as the amino acid sequence of NYAP2 can be confirmed from websites such as UniProt or NCBI. The primary accession number for NYAP2 in UniProt is Q9P242, and the Gene ID in NCBI is 57624. In the Examples described below, NYAP2 corresponding to the above Q9P242 was detected. NYAP2 may be, for example, NYAP2 derived from a human, monkey, mouse, rat, rabbit, dog, or cat.

In embodiments (e.g., (1) to (48) above) of the present invention, the expression level of a protein (e.g., GPC6 or NYAP2) may be tested by methods such as PCR (polymerase chain reaction), antigen-antibody reaction, immunohistochemical staining, or DNA methylation analysis. PCR includes, for example, qPCR. The antigen-antibody reaction includes, for example, Western blotting. DNA methylation analysis includes, for example, analysis of DNA methylation upstream (e.g., a promoter) of a gene (e.g., the GPC6 or NYAP2 gene). A known method may be used as the method for testing the expression level. When the expression level of a protein in a sample from a subject (e.g., glioma cells or a lysate thereof) is low, the degree thereof may be a degree that is significantly reduced compared to the expression level of the protein in a sample from a comparison subject (e.g., an unspecified or general subject with glioma). The degree of this low expression may be, for example, 10, 5, 3, 1, 0.1, or 0.01% or less, or 0%, or may be within a range between any two of these values. This low expression may be, for example, 10-0, 5-0, 1-0, or 0.1-0%. The degree of this low expression may be equal to or less than the mean value of a comparison sample group minus 1 standard deviation. This value may be, for example, -1, -1.5, -2, -2.5, or -3 SD or less, or may be within a range between any two of these values. When the expression level of a protein in a sample from a subject is low, the degree of low expression may be equal to or less than the mean value of a sample group of gliomas obtained from patients other than the subject minus 1 SD. This value may be, for example, -1, -1.5, -2, -2.5, or -3 SD or less, or may be within a range between any two of these values. The sample group of gliomas obtained from patients other than the subject may be, for example, a sample group in which a high expression level of the protein was confirmed in advance, or a sample group in which low sensitivity to BCV was confirmed in advance. When the expression level of a protein in a sample from a subject is low, the degree of low expression may be comparable to that of a sample in which a low expression level of the protein was confirmed in advance, or a sample in which high sensitivity to BCV was confirmed in advance. Such a sample may be, for example, the KALS-1, T98G, and LN-18 cell lines. This comparable degree may be, for example, a difference in expression level of ±30, 20, 10, 5, 3, 2, 1, or 0.1 % or less, or 0%, or may be within a range between any two of these values. This value may be, for example, ±10-0, 5-0, 1-0, or 0.1-0%. When the expression level of a protein in a sample from a subject is low, the degree of low expression may be a markedly low degree compared to a sample with a high expression level of the protein, a sample in which a high expression level of the protein was confirmed in advance, a sample in which the expression level of the protein is not low, or a sample in which low sensitivity to BCV was confirmed in advance. The degree of this markedly low level may be, for example, 10, 5, 3, 1, 0.1, or 0.01% or less, or 0%, or may be within a range between any two of these values. This value may be, for example, 10-0, 5-0, 1-0, or 0.1-0%. The expression level may be evaluated, for example, by the amount of mRNA or the amount of protein. Considering the effect on the function of the protein, it is preferable to evaluate the expression level by the amount of protein. The evaluation of the expression level may include, for example, a step of comparing the expression amount of the subject with a predetermined threshold, or a step of evaluating the expression level as low when the expression amount of the subject is lower than the threshold. The evaluation of the expression level may include a step of evaluating the expression level as low when the expression amount of the subject is significantly reduced compared to the value of a control. In embodiments (e.g., (1) to (48) above or this paragraph) of the present invention, low protein expression may be protein negative. The above embodiments regarding low protein expression can be applied to embodiments of protein negativity. The testing of the protein expression level may be performed, for example, in vitro, ex vivo, or in vivo.

In embodiments (e.g., (1) to (48) above) of the present invention, when the expression level of a protein (e.g., GPC6 or NYAP2) in a sample from a subject (e.g., glioma cells or a lysate thereof) is high, the degree thereof may be a degree that is significantly increased compared to the expression level of the protein in a sample from a comparison subject (e.g., an unspecified or general subject with glioma). The degree of this high expression may be, for example, 1.3, 1.5, 2, 3, 4, 5, 10, 20, 30, 40, 50, 100, or 500 times or more, or may be within a range between any two of these values. This high expression may be, for example, 1.3-500, 2-500, 5-500, or 50-500 times. The degree of this high expression may be equal to or greater than the mean value of a comparison sample group plus 1 standard deviation. This value may be, for example, +1, +1.5, +2, +2.5, or +3 SD or more, or may be within a range between any two of these values. When the expression level of a protein in a sample from a subject is high, the degree of high expression may be equal to or greater than the mean value of a sample group of gliomas obtained from patients other than the subject plus 1 SD. This value may be, for example, +1, +1.5, +2, +2.5, or +3 SD or more, or may be within a range between any two of these values. The sample group of gliomas obtained from patients other than the subject may be, for example, a sample group in which a low expression level of the protein was confirmed in advance, or a sample group in which low sensitivity to BCV was confirmed in advance. When the expression level of a protein in a sample from a subject is high, the degree of high expression may be comparable to that of a sample in which a high expression level of the protein was confirmed in advance, or a sample in which high sensitivity to BCV was confirmed in advance. Such a sample may be, for example, the KALS-1, T98G, and LN-18 cell lines. This comparable degree may be, for example, a difference in expression level of ±30, 20, 10, 5, 3, 2, 1, or 0.1 % or less, or 0%, or may be within a range between any two of these values. This value may be, for example, ±10-0, 5-0, 1-0, or 0.1-0%. When the expression level of a protein in a sample from a subject is high, the degree of high expression may be a markedly high degree compared to a sample with a low expression level of the protein, a sample in which a low expression level of the protein was confirmed in advance, a sample in which the expression level of the protein is not high, or a sample in which low sensitivity to BCV was confirmed in advance. The degree of this markedly high level may be, for example, 1.3, 1.5, 2, 3, 4, 5, 10, 20, 30, 40, 50, 100, or 500 times or more, or may be within a range between any two of these values. This value may be, for example, 1.3-500, 2-500, 5-500, or 50-500 times. The expression level may be evaluated, for example, by the amount of mRNA or the amount of protein. Considering the effect on the function of the protein, it is preferable to evaluate the expression level by the amount of protein. The evaluation of the expression level may include, for example, a step of comparing the expression amount of the subject with a predetermined threshold, or a step of evaluating the expression level as high when the expression amount of the subject is higher than the threshold. The evaluation of the expression level may include a step of evaluating the expression level as high when the expression amount of the subject is significantly increased compared to the value of a control. In embodiments (e.g., (1) to (48) above or this paragraph) of the present invention, high protein expression may be protein positive or protein overexpression. The above embodiments regarding high protein expression can be applied to embodiments of protein positivity. The testing of the protein expression level may be performed, for example, in vitro, ex vivo, or in vivo.

In embodiments (e.g., (1) to (48) above) of the present invention, testing includes, for example, detection or measurement. In embodiments (e.g., (1) to (48) above) of the present invention, the testing reagent may be, for example, a nucleic acid (e.g., a DNA strand or an RNA strand), a peptide (e.g., an oligopeptide or a polypeptide (e.g., a protein)), or a composition containing them. The nucleic acid may be, for example, a primer or a probe. The polypeptide may be, for example, an antibody. The nucleic acid may be, for example, a nucleic acid capable of binding to an upstream or downstream region of a DNA encoding a protein (e.g., GPC6 or NYAP2) (e.g., the GPC6 or NYAP2 gene), or a nucleic acid capable of amplifying the full length or a part of a DNA encoding a protein. The peptide or antibody may be, for example, a peptide or an antibody capable of binding to a protein. An oligopeptide may refer to a peptide having 1 to 20 amino acids. A polypeptide may refer to a peptide having 21 or more amino acids. The antibody may be, for example, a human, mouse, rabbit, rat, hamster, or guinea pig antibody. The antibody may be a chimeric antibody of any two or more of the above organisms. The antibody may be a monoclonal antibody. The testing reagent may be replaced with a detection means. In embodiments (e.g., (1) to (48) above) of the present invention, the testing step may be performed, for example, by PCR, antigen-antibody reaction, immunohistochemical staining, or methylation analysis of the promoter region DNA of a gene (e.g., the GPC6 or NYAP2 gene). The testing may be performed, for example, using the amount of nucleic acid (e.g., gene or mRNA), the amount of protein, DNA methylation, or gene translocation as an indicator. DNA methylation analysis may be performed using a means for detecting methylation or unmethylation of the promoter region of a gene (e.g., a reagent used for methylation-specific PCR, unmethylation-specific PCR, or a pyrosequencing method). DNA methylation analysis may be performed using a primer (e.g., a primer capable of amplifying at least a part of the promoter region (e.g., a primer that can bind to a site upstream or downstream of the promoter region)) or an antibody. The primer may be a methylation-specific or unmethylation-specific primer. The antibody may be an antibody specific to the promoter region of a gene in a methylated or unmethylated state, or a partial region thereof. The above testing reagents may include those used in these detection steps. In embodiments (e.g., (1) to (48) above) of the present invention, the step of testing the expression level may be replaced, for example, with a step of testing the expression or a step of testing for the presence of positivity/negativity.

In embodiments (e.g., (1) to (48) above) of the present invention, the expression level of GPC6 or NYAP2 as a testing target or an indicator may be GPC6 low expression or negative, or NYAP2 high expression or positive. For example, when identifying a glioma patient to be treated with BCV, the identification may be made using GPC6 low expression or negative, or NYAP2 high expression or positive as a testing target or an indicator. This can be expected to improve the probability of a therapeutic effect appearing or to improve the therapeutic effect, compared to when BCV treatment is performed on an unspecified patient. The expression level may be tested qualitatively or quantitatively.

The methods of the embodiments (e.g., (1) to (31) above) of the present invention may include, for example, (i) a step of testing the expression level of GPC6 or NYAP2 of a subject, (ii) a step of identifying the subject as a subject for treatment with BCV based on the expression level of GPC6 or NYAP2 of the subject as an indicator, (iii) a step of identifying a subject having a GPC6-low-expressing glioma as a subject for treatment with BCV, or a step of identifying a subject having a NYAP2-high-expressing glioma as a subject for treatment with BCV, (iv) a step in which it is shown that the subject is or is likely to be sensitive to BCV when the expression level of GPC6 of the subject is low, or (v) a step in which it is shown that the subject is or is likely to be sensitive to BCV when the expression level of NYAP2 of the subject is high. Adopting one or more of these steps is useful for more appropriate BCV treatment (e.g., for providing treatment to a population in which the treatment is highly effective). When two or more steps are adopted, their order is arbitrary and can be determined according to the desired treatment method. These steps may be performed prior to or subsequent to the step of administering BCV, a pharmaceutically acceptable salt thereof, or a solvate thereof, or a chemotherapeutic agent to a subject.

The methods of the embodiments (e.g., (1) to (31) above) of the present invention may include, for example, (i) a step of testing the expression level of EGFR, PTEN, p53, or p16 of a subject, (ii) a step of identifying the subject as a subject for treatment with BCV based on the expression level of EGFR, PTEN, p53, or p16 of the subject as an indicator, or (iii) a step of identifying a subject having a positive, negative, wild-type, or mutant-type glioma of EGFR, PTEN, p53, or p16 as a subject for treatment with BCV. Adopting one or more of these steps is useful for more appropriate BCV treatment. When two or more steps are adopted, their order is arbitrary and can be determined according to the desired treatment method. These steps may be performed prior to or subsequent to the step of administering BCV, a pharmaceutically acceptable salt thereof, or a solvate thereof, or a chemotherapeutic agent to a subject.

In embodiments (e.g., (1) to (31) above) of the present invention, the method may include an assisting method. The assistance may include assistance in examining or determining a subject for treatment, a treatment method, or a diagnostic method. The method may be performed, for example, in vitro, ex vivo, or in vivo, depending on the purpose.

In embodiments (e.g., (1) to (48) above) of the present invention, the step of identifying a subject may include a step of identifying a subject as a subject for treatment. The step of identifying as a subject for treatment may include a step of identifying a subject as a subject for administration of a drug. The step of identifying a subject may include a step of selecting a subject or a step of identifying a subject.

In embodiments (e.g., (1) to (48) above) of the present invention, the sample may include, for example, a biological sample. The sample may include, for example, cells (e.g., glioma cells), a cell lysate, a blood specimen (e.g., plasma, serum, or whole blood), tissue, or a nucleic acid or protein contained in glioma cells. The sample may include, for example, a specimen.

Herein, caused by includes, for example, being the main cause, or being the cause due to past experience. Herein, a broader term includes a narrower term, but they may be described in parallel to clarify that both the broader and narrower concepts are intended.

In one embodiment of the present invention, "significant" may be, for example, a state in which a statistical significance is evaluated using Student's t-test (one-sided or two-sided), and p < 0.05 or p < 0.01. Alternatively, it may be a state in which a substantial difference has occurred.

All publications cited herein are incorporated by reference in their entirety. Herein, "or" is used when "at least one or more" of the items listed in the text can be adopted. The same applies to "or". Herein, when "within a range of two values" is specified, the range includes the two values themselves. Herein, "A to B" includes A and B. Herein, "(1) to (48) above" includes a reference to any one or more of (1), (2), (3), (4), (5), (6), (7), (8), (9), (10), (11), (12), (13), (14), (15), (16), (17), (18), (19), (20), (21), (22), (23), (24), (25), (26), (27), (28), (29), (30), (31), (32), (33), (34), (35), (36), (37), (38), (39), (40), (41), (42), (43), (44), (45), (46), (47), or (48).

While embodiments of the present invention have been described above, these are illustrative embodiments that may be included in the present invention. The present invention is not limited to these and may also adopt various configurations other than those described above. The present invention may also adopt the respective configurations or features described in the above-described embodiments in combination or independently.

### Examples

Hereinbelow, the present invention will be described in more detail with reference to Examples. However, the present invention is not limited to them.

### Example 1: Antitumor effect of BCV

### (1) Test method

A GBM animal model was created by implanting 3 x 10⁵ cells of a U87MG human glioblastoma (GBM) cell line previously transfected with firefly luciferase-GFP into the right striatum of nude mice (2 mm to the right of the bregma on the skull surface and 1 mm anterior to the coronal suture, at a depth of 3 mm from the skull surface).

The study was composed of four groups of 8 to 10 GBM model mice per group. The first group was administered with 10 mg/kg of BCV, the second group with 20 mg/kg of BCV, the third group with 40 mg/kg of BCV, and the fourth group with the vehicle only. BCV and the vehicle were administered intraperitoneally twice weekly for 5 weeks, starting on day 7 post-GBM implantation. During the administration period, to evaluate tumor growth in the brain, bioluminescence imaging (BLI) was performed twice weekly using the IVIS Lumina imaging system, 10 minutes after intraperitoneal administration of 150 mg/kg luciferin under ketamine-xylazine anesthesia.

### (2) Results and evaluation

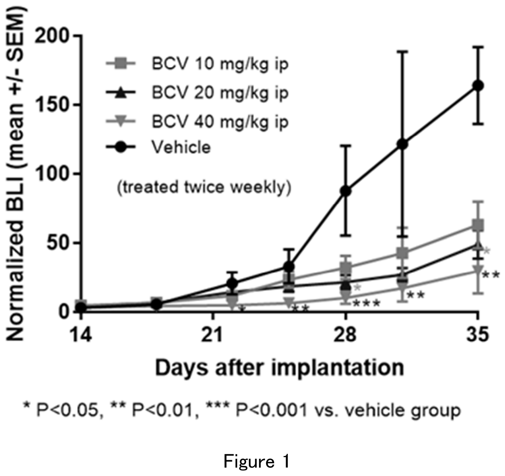

Figure 1 shows the time course of normalized BLI (mean ± standard error), which represents the proliferation of GBM cells in the brain. The normalized BLI was obtained by dividing the BLI during the administration period by the pre-administration BLI for each individual. The 10 mg/kg BCV group showed a tendency to suppress the growth of intracerebral GBM compared to the vehicle group, although the difference was not statistically significant. In the 20 mg/kg BCV group, a significant (P < 0.05) decrease in normalized BLI was observed on days 28 and 35 post-implantation compared to the vehicle group. In the 40 mg/kg BCV group, a significant (P < 0.05 to P < 0.0001) decrease in normalized BLI was observed between days 22 and 35 post-implantation compared to the vehicle group.

In addition, weight loss was observed with the administration of 40 mg/kg BCV. No remarkable systemic toxicity symptoms were observed in the other BCV-treated groups. From the above, it was shown that BCV brings about an excellent effect in the treatment of glioblastoma.

### Example 2: Life-prolonging effect of combination of BCV and TMZ

### (1) Test method

A GBM animal model was created in the same manner as in Example 1. The study was composed of six groups of 9 to 11 GBM model mice per group. The first group was administered with 2 mg/kg of temozolomide (TMZ), the second group with 10 mg/kg of BCV, the third group with 20 mg/kg of BCV, the fourth group with a combination of 2 mg/kg TMZ and 10 mg/kg BCV, the fifth group with a combination of 2 mg/kg TMZ and 20 mg/kg BCV, and the sixth group with the vehicle only. TMZ was orally administered daily for 5 days starting on day 8 post-implantation, and BCV was administered intraperitoneally twice weekly from day 8 to day 38 post-implantation. BLI was evaluated in the same manner as in Example 1, and the survival rate on day 37 post-GBM implantation was examined.

### (2) Results and evaluation

The monotherapy groups of TMZ and 10 mg/kg BCV showed a tendency to suppress the growth of intracerebral GBM compared to the vehicle group, although the difference was not statistically significant. On the other hand, in the combination therapy group of TMZ and 10 mg/kg BCV, a significant (P < 0.01) decrease in normalized BLI was observed on day 28 post-implantation compared to the vehicle group. In the 20 mg/kg BCV monotherapy group, a significant (P < 0.05) decrease in normalized BLI was observed on days 23 and 28 post-implantation compared to the vehicle group, and in the combination therapy group of TMZ and 20 mg/kg BCV, a decrease in normalized BLI with stronger significance (P < 0.05 to P < 0.001) was observed during the same period compared to the vehicle group.

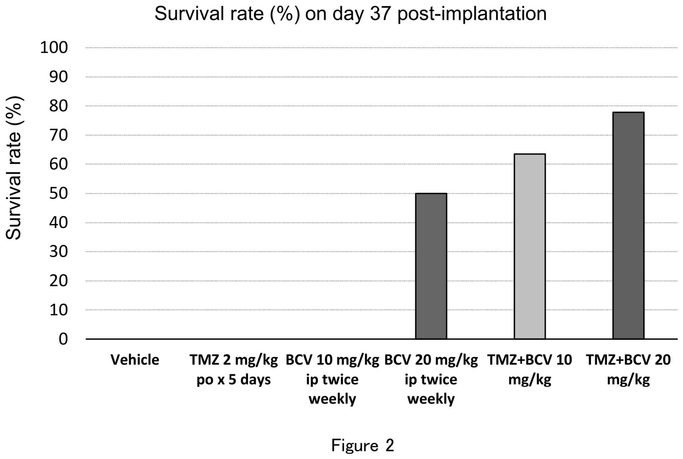

Figure 2 shows the survival rate (%) in each group on day 37 post-GBM implantation. In the vehicle group, the TMZ monotherapy group, and the 10 mg/kg BCV monotherapy group, no individuals survived. On the other hand, the survival rates were 50% in the 20 mg/kg BCV monotherapy group, approximately 64% in the combination therapy group of TMZ and 10 mg/kg BCV, and approximately 78% in the combination therapy group of TMZ and 20 mg/kg BCV.

In addition, no remarkable systemic toxicity symptoms were observed in any of the BCV monotherapy and combination therapy groups. From the above, a synergistic life-prolonging effect was observed with the combination administration of BCV and TMZ. Furthermore, since the life-prolonging effect of the combination administration of 10 mg/kg BCV and TMZ was close to that of the combination administration of 20 mg/kg BCV and TMZ, it was shown that the regimen of combination administration of 10 mg/kg BCV and TMZ is superior from the viewpoint of exerting a sufficient effect while suppressing the dosage.

### Example 3: Antitumor effect of combination of BCV, TMZ, and radiation therapy

### (1) Test method

A GBM animal model was created in the same manner as in Example 1. The study was composed of three groups of 10 GBM model mice per group. The first group was treated with a combination of 2 mg/kg TMZ and 1.5 Gray (Gy) radiation therapy (RT) (TMZ+RT therapy), the second group with a combination of 10 mg/kg BCV with TMZ+RT therapy (BCV+TMZ+RT therapy), and the third group with the vehicle only. TMZ was orally administered daily for 5 days starting on day 8 post-implantation, RT was performed by local brain irradiation using a cesium gamma-ray irradiator during the same period, and BCV was administered intraperitoneally twice weekly from day 8 to day 49 post-implantation. BLI was evaluated in the same manner as in Example 1.

### (2) Results and evaluation

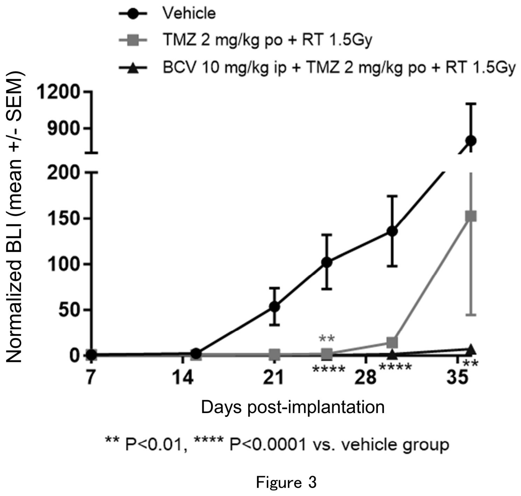

Figure 3 shows the time course of normalized BLI (mean ± standard error), which represents the proliferation of GBM cells in the brain. In the TMZ+RT therapy group, a significant (P < 0.01) decrease in normalized BLI was observed on day 25 post-implantation compared to the vehicle group. In the BCV+TMZ+RT therapy group, a decrease in normalized BLI with stronger significance (P < 0.01 to 0.0001) was observed over a longer period (from day 25 to day 36) post-implantation compared to the vehicle group.

In addition, no remarkable systemic toxicity symptoms were observed in the BCV+TMZ+RT therapy group. From the above, it was shown that BCV+TMZ+RT therapy brings about an excellent effect in the treatment of glioblastoma.

### Example 4: Antitumor effect and life-prolonging effect on glioblastoma with unmethylated MGMT gene promoter

### (1) Test method

An orthotopic GBM PDX model was created in the same manner as in Example 1, using GBM6 cells derived from a GBM patient, which had been previously transfected with GFP. GBM6 cells are cells in which the promoter of the DNA repair gene MGMT, which inhibits the antitumor effect of alkylating agents, is not methylated. The study was composed of two groups of 9 to 11 PDX mice per group. The first group was administered with 20 mg/kg of BCV, and the second group with the vehicle only. BCV was administered intraperitoneally twice weekly from day 6 to day 30 post-implantation. BLI was evaluated in the same manner as in Example 1, and the survival rate on day 27 post-GBM implantation was examined.

### (2) Results and evaluation

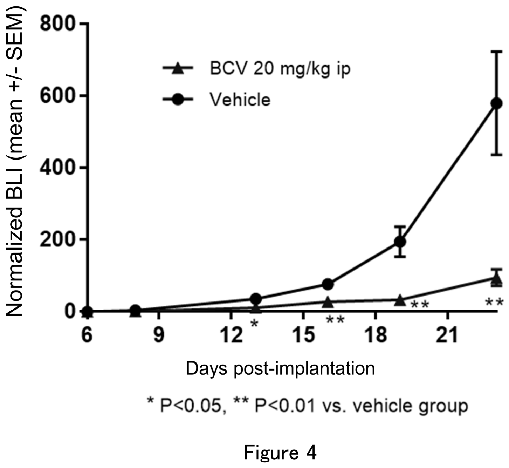

Figure 4 shows the time course of normalized BLI (mean ± standard error), which represents the proliferation of GBM cells in the brain, in PDX mice using GBM6 cells. With the administration of 20 mg/kg BCV, a significant (P < 0.05 to P < 0.01) decrease in normalized BLI was observed between days 13 and 23 post-implantation compared to the vehicle group.

In the vehicle group, no individuals survived on day 27 post-GBM implantation. On the other hand, 60% of the individuals survived in the 20 mg/kg BCV group.

In addition, no remarkable systemic toxicity symptoms were observed in the BCV-treated group. It has been reported that the gene promoter of MGMT (O6-methylguanine-DNA methyltransferase) is unmethylated in GBM6 (Int J Radiat Oncol Biol Phys 2009, 75: 212-219). It is also known that when the expression of MGMT is enhanced due to the unmethylation of the MGMT gene promoter, the alkylating effect of TMZ is attenuated, and cells acquire resistance to TMZ (Cancer Drug Resist 2021;4:17-43). In fact, it has been suggested that the effect of TMZ is significantly attenuated in GBM6 compared to other PDXs in which the MGMT gene promoter is methylated (Int J Radiat Oncol Biol Phys 2009, 75: 212-219). As described above, since tumor shrinkage and survival extension were observed in the GBM6 PDX model, it was shown that BCV brings about an excellent therapeutic effect also in glioblastoma with an unmethylated MGMT gene promoter.

### Example 5: PK study of BCV

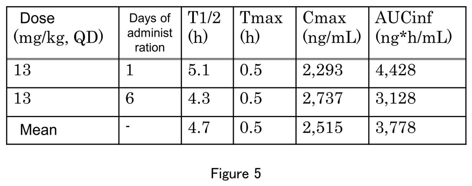

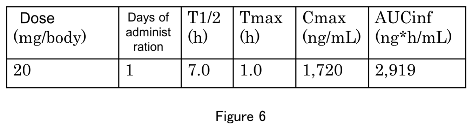

Female C57BL/6 mice were administered intraperitoneally with BCV at 13 mg/kg (n=3) and, after continuous administration of the same dose for 6 days, blood samples were collected at 0.5, 1, 2, 4, 8, 12, and 24 hours. The plasma BCV concentration obtained from K2EDTA-treated samples was quantified by a previously reported MC-MS/MS method (Biomed Chromatogr. 2021 35:e5061). Figure 5 shows the results of pharmacokinetic parameters obtained by non-compartmental analysis using WinNonlin. From the average values of the results on day 1 and after 6 days of continuous administration, the drug exposure obtained when BCV was administered intraperitoneally to mice at 10 mg/kg was estimated to be 1,935 ng/mL (Cmax) and 2,906 ng*h/mL (AUCinf). In addition, Figure 6 shows the pharmacokinetic parameters of intravenous administration of BCV to humans obtained in a Phase 1 clinical trial (from CMX001-125 study, ID WEEK Congress, 2018 Poster#1421). From these results, the drug exposure obtained by intraperitoneal administration of 10 mg/kg BCV in mice was estimated to be comparable to the drug exposure obtained by intravenous administration of 20 mg (1h-infusion) in humans. Based on the above, the intraperitoneal administration of 10 mg/kg BCV, 20 mg/kg BCV, and 40 mg/kg BCV to mice in Examples 1 to 4 (including monotherapy or combination therapy) can be converted to intravenous administration of 20 mg/body BCV, 40 mg/body BCV, and 80 mg/body BCV to humans, respectively.

### Example 6: Cytotoxicity test against human GBM cell lines

The in vitro cytotoxicity of BCV against various human GBM cell lines shown in Table 1 was evaluated by the CellTiter-GloTM (CTG) assay (Promega, Wisconsin, USA). The CTG assay measures the number of viable cells in culture by quantifying intracellular adenosine triphosphate (ATP), an indicator of metabolic activity, by bioluminescence. Specifically, various concentrations of BCV were added to 96-well plates in which GBM cells were plated at a concentration of 2 x 10³ cells in each medium shown in Table 1, and after 5 days of culture at 37°C and 5% CO₂, the cells were treated with CTG reagent and lysed. The fluorescence signal was measured using an Envision^{™} plate reader (PerkinElmer, Connecticut, USA). Cytotoxicity was evaluated as a percentage of the control fluorescence signal. A dose-response curve was generated by plotting the percentage of cytotoxicity against the drug concentration using GraphPad Prism (GraphPad Software, Massachusetts, USA). Furthermore, the 50% inhibitory concentration (IC₅₀ value) of BCV against various GBM cell lines was determined to evaluate the cytotoxic effect. All assays were performed in duplicate.

**[Table 1]**

| Table 1: Various human GBM cell lines and their culture media | |
|---|---|
| Cell line | Medium |
| A-172 | DMEM¹⁾ + 10% FBS²⁾ |
| AM-38 | EMEM³⁾ +20% FBS |
| KALS-1 | RPMI⁴⁾ 1640 +10% FBS |
| KNS-42 | DMEM +10% FBS |
| LN-18 | DMEM +10% FBS |
| LN-229 | DMEM +10% FBS |
| SNU-466 | RPMI 1640 + 10% FBS |
| SNU-1105 | RPMI 1640 +10% FBS |
| T98G | EMEM + 10% FBS |
| U-87MG | EMEM +10% FBS |
| U-138 MG | EMEM + 10% FBS |

| | |
|---|---|
| ¹⁾ Dulbecco's modified Eagle's medium ²⁾ Fetal bovine serum ³⁾ Eagle's minimal essential medium ⁴⁾ Roswell Park Memorial Institute | |

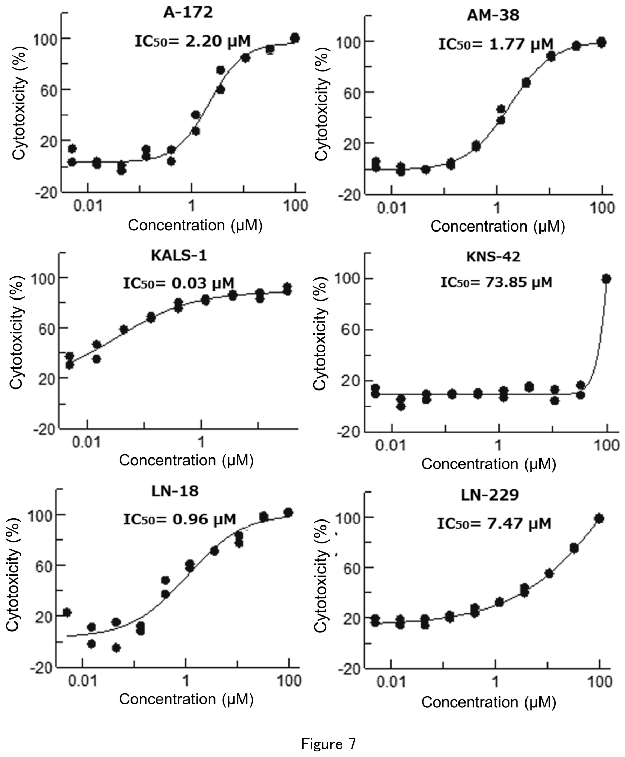

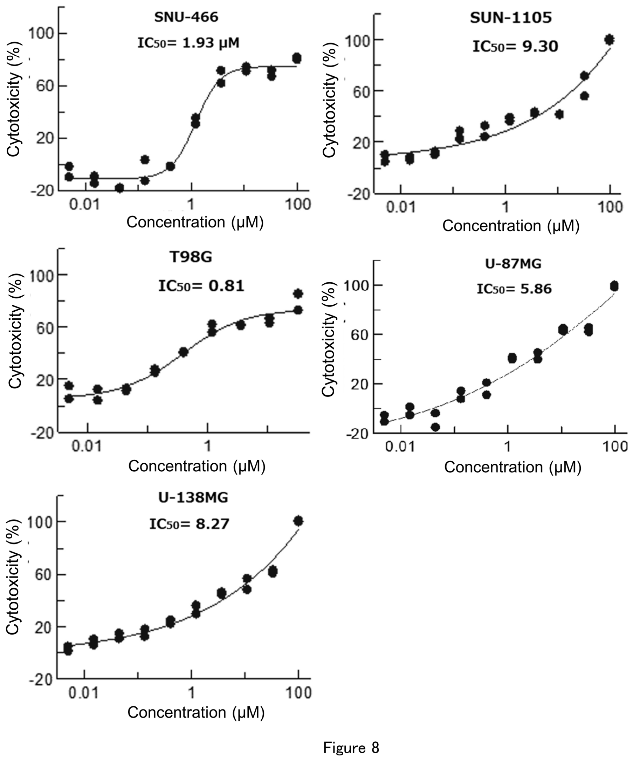

The results are shown in Figures 7 and 8. BCV showed dose-dependent cytotoxicity against all GBM cell lines, and the median IC₅₀ value for all cell lines was 2.2 µM. As shown in Figure 6 of Example 5, the maximum blood BCV concentration upon intravenous administration of a clinical dose of 20 mg was 3.1 µM (1,720 ng/mL). This revealed that the majority of the human GBM cell lines used in the study showed IC₅₀ values at concentrations below the maximum clinical concentration.

Resistance mechanisms to chemotherapy in GBM include the expression of O⁶-methylguanine-DNA methyltransferase (MGMT), a DNA repair enzyme (Cancer Drug Resist 2021;4:17-43). The MGMT expression levels of the GBM cell lines used in the study were collected from an open database (The Human Protein Atlas) and evaluated together with the IC₅₀ values of BCV (listed in ascending order) (Table 2). As a result, all highly sensitive cell lines (KALS-1, T98G, and LN-18) that showed IC₅₀ values of µM or less for BCV had high MGMT expression. In addition, among the cell lines that showed IC₅₀ values exceeding the maximum clinical blood concentration (U-87MG, LN-229, U-138MG, SNU-1105, KNS-42), only U-138MG had high MGMT expression. These findings indicated that high expression of MGMT in GBM cells does not contribute to BCV resistance.

**[Table 2]**

| Table 2: IC₅₀ values and MGMT¹⁾ gene expression status in various human GBM cell lines | | |
|---|---|---|
| Cell line | **IC₅₀ (µM)** | **MGMT¹⁾ (nTPM²⁾)** |
| KALS-1 | **0.03** | 21.1 |
| T98G | **0.81** | 17.1 |
| LN-18 | **0.96** | 20.2 |
| AM-38 | **1.77** | 0.1 |
| SNU-466 | **1.93** | 30.8 |
| A-172 | **2.20** | 0.0 |
| U-87MG | **5.86** | 0.1 |
| LN-229 | **7.47** | 0.0 |
| U-138 MG | **8.27** | 14.0 |
| SNU-1105 | **9.30** | 0.1 |
| KNS-42 | **73.85** | 0.0 |

| | | |
|---|---|---|
| ¹⁾ O⁶-methylguanine-DNA methyltransferase ²⁾ Normalized transcripts per million | | |

### Example 7: Forced expression of MGMT gene in MGMT-negative GBM cell line

To further clarify the effect of MGMT expression on sensitivity to BCV, the MGMT gene was forcibly expressed in the U-87MG cell line using a lentivirus as a vector. Specifically, the DNA sequence of human MGMT was cloned into a pLVX-Puro lentiviral vector containing a puromycin resistance gene. HEK293T cells were plated in 10 cm dishes at a density of 3.0 x 10⁶ cells and cultured at 37°C, 5% CO₂ for 24 hours until they reached 50-60% confluence. 4 µg of pLVX-Puro-MGMT, 4 µg of pΔ8.9, and 2 µg of pVSVG were diluted with 500 µL of OPTI-MEM^{™} (Thermo Fisher Scientific), and 40 µL of Invitrogen^{™} Lipofectamine^{™} 2000 (Thermo Fisher Scientific) was diluted with 500 µL of OPTI-MEM^{™}. Then, this plasmid mixture was added to the transfection reagent, mixed, and added dropwise to the 10 cm dish containing HEK293T cells and stirred. After 48 hours of culture, the cell culture medium containing the modified lentivirus was collected, and cell debris was removed by centrifugation at 3,000 rpm for 10 minutes. After further filtration through a 0.45 µM filter, it was dispensed into vials. After trypsin treatment of U-87MG cells, the cell density was adjusted to 2 x 10⁵ /ml. 2 ml/well of the cell suspension was dispensed into a 6-well plate, and the cells were cultured at 37°C, 5% CO₂. After 24 hours, 1 ml of the virus-containing solution was added. After 24 hours of culture of the cell plate at 37°C, 5% CO₂, screening was performed for 7 days with a final concentration of 1 µg/ml puromycin, and pooled cells (U-87MG/MGMT) were collected. The MGMT expression level was evaluated using quantitative polymerase chain reaction (PCR) and Western blotting.

Quantitative PCR using U-87MG/MGMT pooled cells was performed in triplicate using an Applied Biosystems^{™} QuantStudio^{™} (Thermo Fisher Scientific) to acquire data. The following primers were used: for the MGMT gene, the forward primer 5'-GCAATGAGAGGCAATCCTG-3' (SEQ ID NO: 1) and the reverse primer 5'-CCAGAAGCCATTCCTTCAC-3' (SEQ ID NO: 2); and for the internal control gene glyceraldehyde-3-phosphate dehydrogenase (GAPDH), the forward primer 5'-CCACTCCTCCACCTTTG-3' (SEQ ID NO: 3) and the reverse primer 5'-CACCACCCTGTTGCTGT-3' (SEQ ID NO: 4). RNA expression was quantified by the ΔΔCt method. Specifically, the ΔCt value of gene mRNA expression was the difference from the Ct value of the internal control GAPDH, and the ΔΔCt value was calculated by subtracting the ΔCt value of the U87MG group from each ΔCt value. The expression ratio was calculated using the formula ^{2^-ΔΔCt} . The results are shown in Table 3. Compared to the parent strain U-87MG, a remarkable enhancement of MGMT gene expression was observed in U-87MG/MGMT pooled cells.

**[Table 3]**

| Table 3: MGMT gene expression in U-87MG/MGMT pooled cells by quantitative PCR | | |
|---|---|---|
| Triplicate | U87MG | U-87MG/MGMT pooled cell |

| **MGMT Ct¹⁾ value** | | |
|---|---|---|
| 1 | Undetermined | 11.590 |
| 2 | Undetermined | 11.655 |
| 3 | 38.974 | 11.544 |
| Average | 38.974 | 11.596 |

| **GAPDH Ct value** | | |
|---|---|---|
| 1 | 16.013 | 15.145 |
| 2 | 15.875 | 14.861 |
| 3 | 15.644 | 14.661 |
| Average | 15.844 | 14.889 |

| **Relative quantification** | | |
|---|---|---|
| ΔCt | 23.130 | -3.292 |
| ΔΔCt | 0.000 | -26.422 |
| **2^-ΔΔCt** | **1.00** | **8.99E+07** |

| | | |
|---|---|---|
| ¹⁾Threshold cycle | | |

For Western blotting, whole-cell lysates were separated by SDS-PAGE using 4-15% Mini-PROTEAN^{™} TGX Stain-Free^{™} Protein Gel (Bio-Rad Laboratories, California, USA) and transferred to a PVDF blotting membrane (Bio-Rad Laboratories). After blocking with 5% bovine serum albumin (Sigma-Aldrich, Darmstadt, Germany), the membrane was gently shaken overnight at 4°C in a solution containing primary antibodies (anti-MGMT antibody: Invitrogen^{™} catalog number MA5-13506, Thermo Fisher Scientific; anti-α-Tubulin antibody; catalog number 2125S, Cell Signaling Technology, Tokyo, Japan). After exposure to an HRP-conjugated anti-mouse antibody (catalog number c-516102, Santa Cruz, California, USA), chemiluminescent detection was performed using the SuperSignal^{™} Western Blot Substrate Kit (Thermo Fisher Scientific), and imaging was performed with an ImageQuant^{™} LAS 4000 (GE, California, USA).

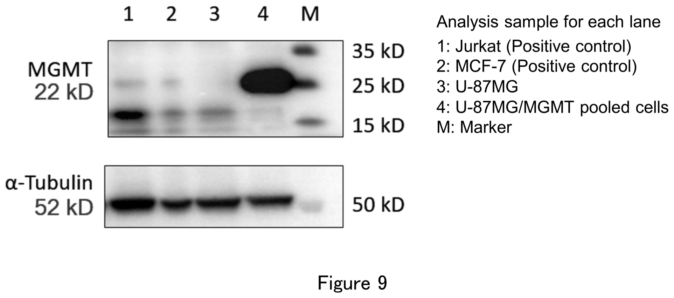

The results are shown in Figure 9. Compared to the parent strain U-87MG, the expression level of MGMT was enhanced in U-87MG/MGMT pooled cells.

Monoclonal cells of the U-87MG cell line with forced expression of the MGMT gene were generated from U-87MG/MGMT pooled cells using the limiting dilution method in a 96-well plate. After treating U-87MG/MGMT pooled cells with 0.25% trypsin, suspensions of 100, 150, and 200 cells/10 mL were prepared and dispensed into a 96-well plate. The cells were cultured at 37°C, 5% CO₂ until single clones grew. MGMT expression in U-87MG/MGMT monoclonal cells was identified by quantitative PCR and Western blotting in the same manner as described above.

Table 4 shows the MGMT gene expression in 12 types of U-87MG/MGMT monoclonal cells by quantitative PCR. No expressed gene was detected (undetermined) in the parent strain U-87MG, but overexpression of the MGMT gene was observed in all monoclonal cells.

**[Table 4]**

| Table 4: MGMT gene expression in 12 types of U-87MG/MGMT monoclonal cells by quantitative PCR | | | | | | | |
|---|---|---|---|---|---|---|---|
| | **MGMT Ct value** | | | | | | |
| Triplicate | U87MG | MGMT-A3-1 | MGMT-A3-3 | MGMT-A8-1 | MGMT-B6-3 | MGMT-C2-2 | MGMT-D5-3 |
| 1 | Undetermined | 13.326 | 13.731 | 12.049 | 12.745 | 12.148 | 12.438 |
| 2 | Undetermined | 13.046 | 13.750 | 12.014 | 12.647 | 12.254 | 12.257 |
| 3 | Undetermined | 13.266 | 13.947 | 12.231 | 12.608 | 12.223 | 12.362 |
| Average Ct value | #DIV/0! | 13.212 | 13.809 | 12.098 | 12.667 | 12.208 | 12.352 |

| | **GAPDH Ct value** | | | | | | |
|---|---|---|---|---|---|---|---|
| Triplicate | U87MG | MGMT-A3-1 | MGMT-A3-3 | MGMT-A8-1 | MGMT-B6-3 | MGMT-C2-2 | MGMT-D5-3 |
| 1 | 13.098 | 13.088 | 13.340 | 12.833 | 12.829 | 13.010 | 13.285 |
| 2 | 13.037 | 12.994 | 13.332 | 13.246 | 12.843 | 12.993 | 12.882 |
| 3 | 13.160 | 12.984 | 13.426 | 12.791 | 12.757 | 12.980 | 13.049 |
| Average Ct value | 13.098 | 13.022 | 13.366 | 12.957 | 12.810 | 12.995 | 13.072 |
| **ΔCt** | **#DIV/0!** | **0.190** | **0.443** | **-0.859** | **-0.143** | **-0.786** | **-0.720** |
| | | | | | | | |

| | **MGMT Ct value** | | | | | | |
|---|---|---|---|---|---|---|---|
| Triplicate | MGMT-E7-3 | MGMT-E12-1 | MGMT-F6-3 | MGMT-F11-2 | MGMT-G11-2 | MGMT-H3-3 | |
| 1 | 12.203 | 12.334 | 11.982 | 12.363 | 12.960 | 13.127 | |
| 2 | 12.175 | 12.524 | 12.023 | 12.371 | 13.006 | 12.969 | |
| 3 | 12.203 | 12.379 | 12.004 | 12.448 | 12.893 | 12.967 | |
| Average Ct value | 12.194 | 12.412 | 12.003 | 12.394 | 12.953 | 13.021 | |

| | **GAPDH Ct value** | | | | | | |
|---|---|---|---|---|---|---|---|
| Triplicate | MGMT-E7-3 | MGMT-E12-1 | MGMT-F6-3 | MGMT-F11-2 | MGMT-G11-2 | MGMT-H3-3 | |
| 1 | 12.540 | 12.396 | 12.743 | 13.277 | 12.401 | 13.139 | |
| 2 | 12.592 | 12.177 | 12.612 | 13.283 | 12.587 | 12.909 | |
| 3 | 12.636 | 12.383 | 12.876 | 12.984 | 12.551 | 13.044 | |
| Average Ct value | 12.589 | 12.319 | 12.744 | 13.181 | 12.513 | 13.031 | |
| **ΔCt** | **-0.396** | **0.094** | **-0.741** | **-0.787** | **0.439** | **-0.010** | |

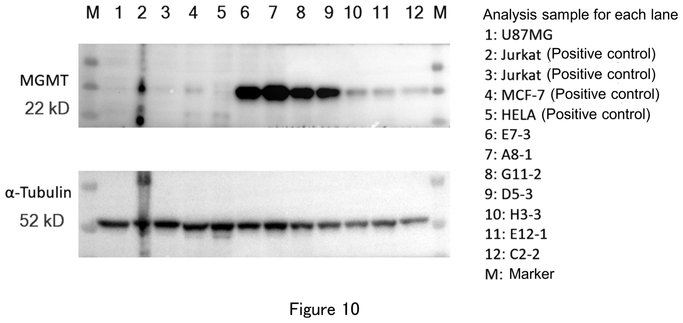

MGMT protein expression in 12 types of U-87MG/MGMT monoclonal cells was evaluated by Western blotting, and Western blotting was re-performed on the 7 types of U-87MG/MGMT monoclonal cells with high expression levels. MGMT protein expression is shown in Figure 10. Compared to the parent strain U-87MG, overexpression of MGMT protein was observed in all monoclonal cells. Among them, E7-3 and A8-1 cells, which had remarkably high MGMT expression levels, were used for the BCV cytotoxicity assay.

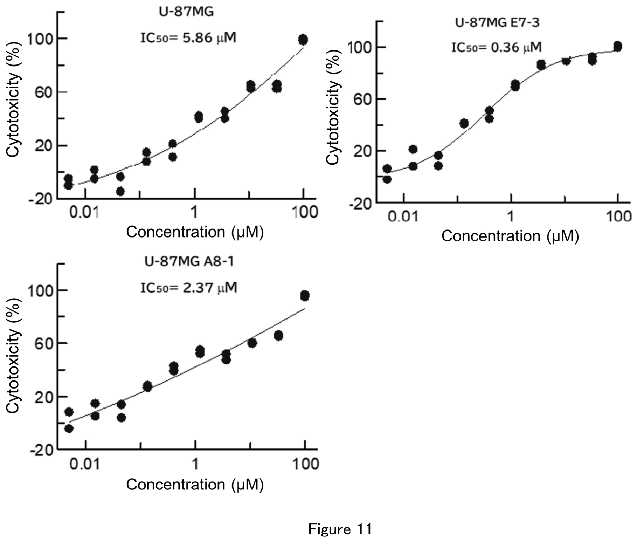

Figure 11 shows the results of the cytotoxicity test performed in the same manner as in Example 6. The sensitivity of U-87MG/MGMT monoclonal cells E7-3 and A8-1 to BCV was 16.28 times and 2.47 times higher, respectively, than that of the parent strain U-87MG cells.

### Example 8: Whole transcriptome sequencing analysis

Whole transcriptome sequencing was performed on the same 11 human GBM cell lines as in Example 6. Each cell line was cultured until the cell number exceeded 5 x 10⁶ and reached 70-90% confluence, and then lysed with the RNA extraction reagent Invitrogen^{™} TRIzol^{™} (Thermo Fisher Scientific). The cell lysates were analyzed using a HiSeq^{™} system (Illumina, California, USA) with paired-end 150 bp reads, 6 Gb/sample, in duplicate. Differential gene expression analysis between the three BCV-highly sensitive cell line groups (KALS-1, T98G, and LN-18) and the remaining eight cell line groups was performed using DESeq2 (Bioconductor open-source software), and a volcano plot was used to show a scatter plot of the gene expression ratio and statistical significance (p-value) between the groups.

The results are shown in Figure 12. The glypican 6 gene (GPC6; expression ratio [log₂] = -6.39, significance [-log₁₀ p-value] = 2.46E-51) was found to be the gene with the most statistically significant decrease in expression in the highly sensitive cell line group. At the same time, Neuronal Tyrosine-Phosphorylated Phosphoinositide-3-Kinase Adaptor 2 (NYAP2; expression ratio [log₂] = 9.15, significance [-log₁₀ *p*-value] = 4.05E-32) was found to be the gene with the most statistically significant increase in expression, and it was shown to be an indicator for predicting high sensitivity, similar to GPC6. In addition, the gene groups described in the figure can be complementary indicators for predicting high sensitivity.

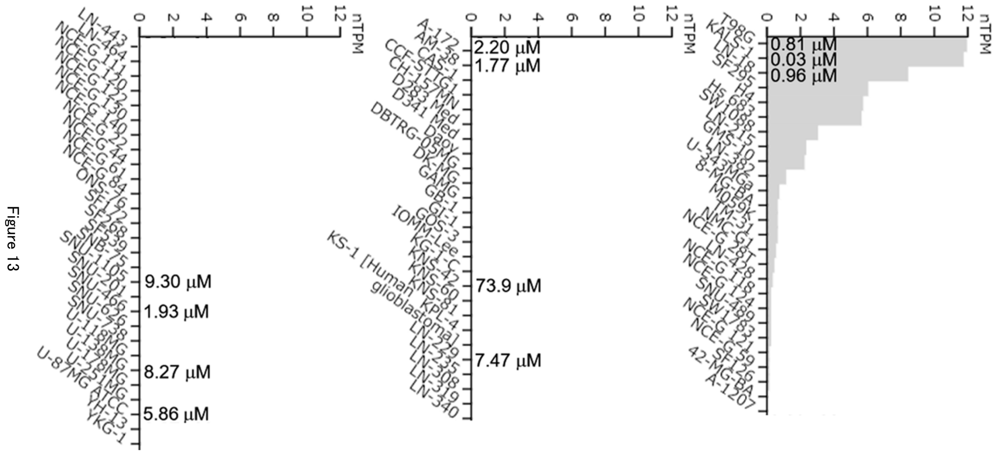

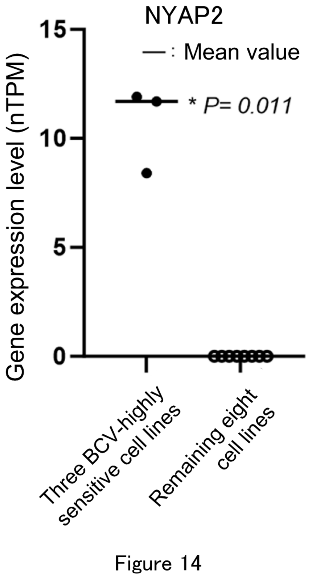

The NYAP2 gene expression levels and IC₅₀ values for BCV of the 11 human GBM cell lines used in this study were compared with other brain tumor cell lines registered in an open database (The Human Protein Atlas). Figure 13 shows the NYAP2 gene expression levels (nTPM) in 80 human brain tumor cell lines in descending order. For the 11 GBM cell lines used in this study, the IC₅₀ values are also shown. It was revealed that the three GBM cell line groups highly sensitive to BCV (KALS-1, T98G, and LN-18) are also the cell lines with the highest NYAP2 gene expression. The NYAP2 gene expression level of these three highly sensitive cell lines was significantly (P = 0.011) higher than that of the remaining eight cell lines (Figure 14).

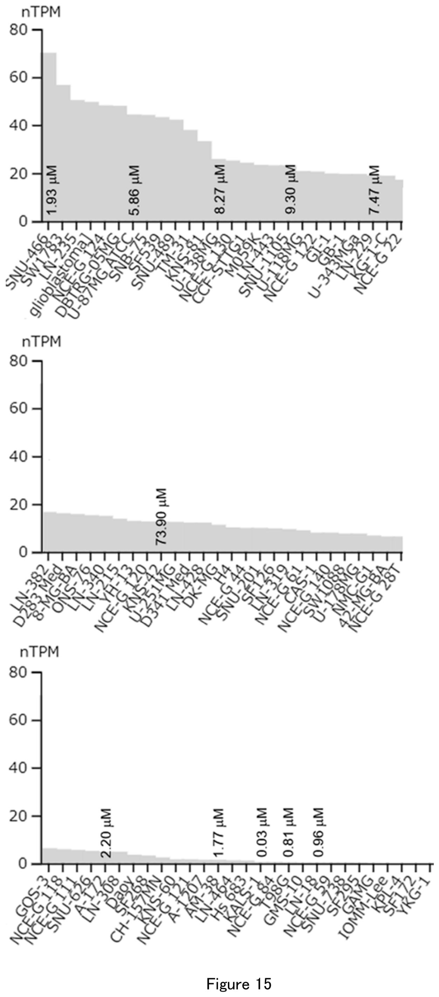

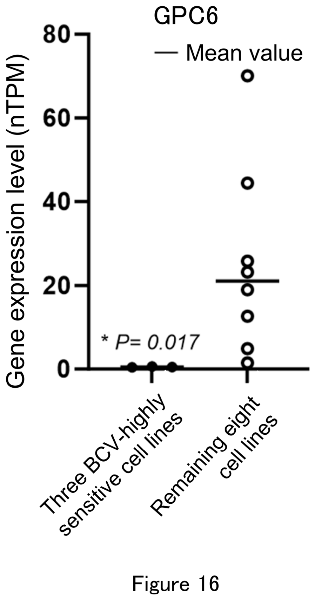

Similarly, the relationship between the expression level of GPC6 and the IC₅₀ value for BCV was confirmed using an open database (The Human Protein Atlas). Figure 15 shows the GPC6 gene expression levels (nTPM) in 80 human brain tumor cell lines in descending order. For the 11 GBM cell lines used in this study, the IC₅₀ values are also shown. It was revealed that the three GBM cell line groups highly sensitive to BCV (KALS-1, T98G, and LN-18) are also the cell lines with extremely low GPC6 gene expression. The GPC6 gene expression level of these three highly sensitive cell lines was significantly (P = 0.017) lower than that of the remaining eight cell lines (Figure 16).

From Example 8, it was revealed that BCV sensitivity in GBM is associated with the expression level of GPC6 or NYAP2. Specifically, it was shown that when the expression level of GPC6 is low, the BCV sensitivity of GBM is high. It was also shown that when the expression level of NYAP2 is high, the BCV sensitivity of GBM is high. Therefore, it is considered that more appropriate BCV treatment can be implemented by predicting sensitivity using the expression level of GPC6 or NYAP2 as an indicator. For example, BCV can be administered to patients whose BCV sensitivity is examined using the expression level of GPC6 or NYAP2 as an indicator and who are predicted to have high sensitivity (i.e., patients with a low expression level of GPC6 or patients with a high expression level of NYAP2). This can be expected to improve the probability of a therapeutic effect appearing or to improve the therapeutic effect, compared to when administered to unspecified patients.

### Example 9: In vivo study using an additional PDX model

### (1) Test method

An orthotopic GBM PDX model was created in the same manner as in Example 1, using GBM39 cells derived from a GBM patient, which had been previously transfected with firefly luciferase-GFP. The study was composed of two groups of 10 PDX mice per group. The first group was administered with 20 mg/kg of BCV, and the second group with the vehicle only. BCV was administered intraperitoneally twice weekly from day 5 to day 28 post-implantation. BLI was evaluated in the same manner as in Example 1, and survival analysis was performed by the Kaplan-Meier method.

### (2) Results and evaluation

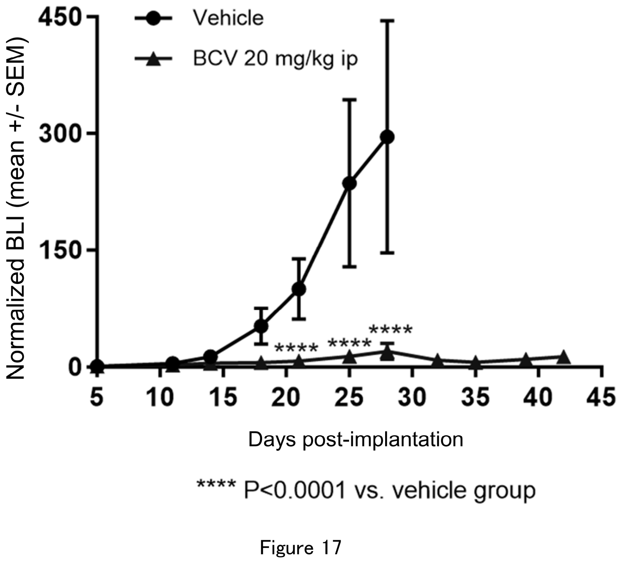

Figure 17 shows the time course of normalized BLI (mean ± standard error), which represents the proliferation of GBM cells in the brain, in PDX mice using GBM39 cells. In the 20 mg/kg BCV-treated group, a significant (P < 0.0001) decrease in normalized BLI was observed between days 21 and 28 post-implantation compared to the vehicle group.

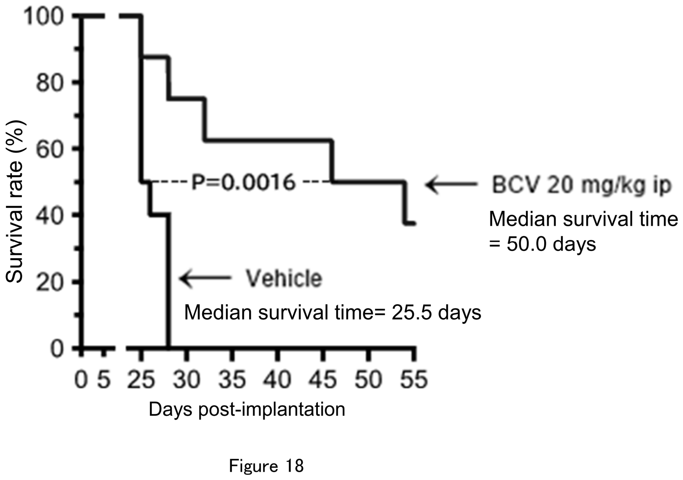

Figure 18 shows the Kaplan-Meier curve plotting the cumulative survival rate after GBM39 cell implantation. In the vehicle group, the median survival time after GBM implantation was 25.5 days. On the other hand, in the 20 mg/kg BCV group, 65% of the individuals were alive on day 33 post-GBM implantation, and the median survival time was 50.0 days (P < 0.01).

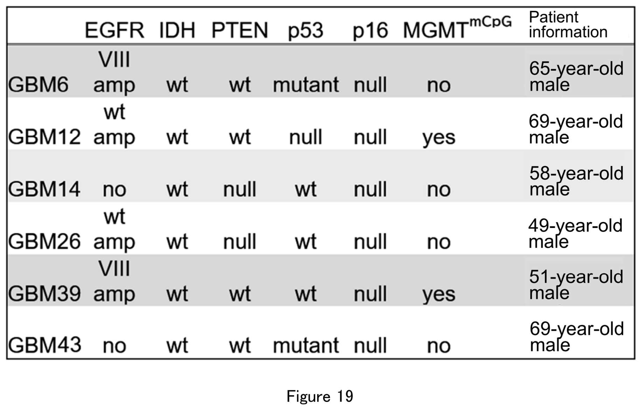

It is known that PDX models retain the microenvironment and tissue structure of patient tumors and have a high clinical effect predictive ability of 30% to 80% compared to other screening systems (Cancer Discov 2014, 4:998-1013). Similar to the PDX model using GBM6 cells in Example 4, BCV also showed an antitumor effect and a life-prolonging effect in the GBM39 PDX model in this example. Therefore, the clinical effect of BCV on GBM is predicted to be 30% to 80% by multiplying the success rate in the two PDX models to date, i.e., 100%, by the clinical effect predictive ability of 0.3 or 0.8 in the PDX model. Figure 19 shows the main genomic characteristics of six types of patient-derived GBM cells (Clin Cancer Res 2020, 26:1097-1104). It is noteworthy that BCV was effective against GBM6 and GBM39 PDX, which have overexpression of EGFR, known as a driver gene mutation in the majority of GBM patients (Cell 2013, 155:462-477). Furthermore, the fact that it was effective regardless of the presence or absence of different p53 mutations or MGMT gene methylation in these two types of PDX indicates that BCV is widely effective for GBM patients with diverse genomic characteristics.

### Example 10: In vivo study using additional PDX models

### (1) Test method

Orthotopic GBM PDX models are created in the same manner as in Example 1, using GBM12, GBM14, GBM26, or GBM43 cells derived from GBM patients, which have been previously transfected with GFP. The study is composed of two groups of 10 PDX mice per group. The first group is administered with 20 mg/kg of BCV, and the second group with the vehicle only. BCV is administered intraperitoneally twice weekly from day 5 to day 28 post-implantation. BLI is evaluated in the same manner as in Example 1, and survival analysis is performed by the Kaplan-Meier method.

### (2) Results and evaluation

The time course of normalized BLI (mean ± standard error), which represents the proliferation of GBM cells in the brain, in PDX mice using GBM12, GBM14, GBM26, or GBM43 cells is examined. The 20 mg/kg BCV-treated group shows a significant decrease in normalized BLI compared to the vehicle group.

The cumulative survival rate after implantation of GBM12, GBM14, GBM26, or GBM43 cells is examined. The 20 mg/kg BCV-treated group shows a longer survival period compared to the vehicle group. No remarkable systemic toxicity symptoms are observed in the BCV-treated group.

As described in the examples above, it was shown that BCV exhibits an excellent effect in the treatment of glioblastoma. Although several research reports on BCV have been published in the past, there are no reports demonstrating a therapeutic effect in animals with glioma. In addition, past research reports have not shown that a dose of BCV suitable for treatment is effective when targeting glioma. BCV is known to have an antiviral effect, but its mechanism of action is the inhibition of viral replication by inhibiting virus-derived DNA polymerase, which is considered to be different from the mechanism of action against solid cancers such as glioblastoma. The results of glioblastoma treatment brought about by BCV can be said to be unexpected results from the prior art.

In the above examples, the following features were shown for the treatment of glioblastoma with BCV. Since a therapeutic effect was observed with low-dose BCV administration, it was shown that BCV administration can provide a treatment with an excellent balance between the therapeutic effect for glioblastoma and the suppression of systemic toxicity. It was also shown that a high life-prolonging effect can be obtained with low doses of drugs by the combination of BCV and TMZ. In addition, from the results of examining the effect of MGMT expression, it was shown that high expression of MGMT in glioblastoma does not contribute to BCV resistance. That is, it was shown that BCV is also effective against glioblastoma that is resistant to alkylating agents. In addition, from the results of examining the effect on glioblastoma with different genomic characteristics, it was shown that BCV is widely effective for patients with diverse genomic characteristics.

Hereinabove, the present invention has been described based on the Examples. It will be understood by those skilled in the art that the Examples are for illustrative purposes only and that various modifications are possible and such modifications are also within the scope of the present invention.

## Claims

1. A pharmaceutical composition for treatment of glioma, comprising brincidofovir, a pharmaceutically acceptable salt thereof, or a solvate thereof.

2. The pharmaceutical composition according to claim 1, wherein the glioma is glioblastoma.

3. The pharmaceutical composition according to claim 1 or 2, comprising brincidofovir.

4. The pharmaceutical composition according to any one of claims 1 to 3, wherein in the treatment, brincidofovir, a pharmaceutically acceptable salt thereof, or a solvate thereof, and a chemotherapeutic agent are used in combination.

5. The pharmaceutical composition according to claim 4, wherein the chemotherapeutic agent is temozolomide, a pharmaceutically acceptable salt thereof, or a solvate thereof.

6. The pharmaceutical composition according to any one of claims 1 to 5, wherein in the treatment, (a) brincidofovir, a pharmaceutically acceptable salt thereof, or a solvate thereof, (b) temozolomide, a pharmaceutically acceptable salt thereof, or a solvate thereof, and (c) radiation therapy are used in combination.

7. The pharmaceutical composition according to any one of claims 1 to 5, wherein in the treatment, brincidofovir, a pharmaceutically acceptable salt thereof, or a solvate thereof, and radiation therapy are used in combination.

8. The pharmaceutical composition according to any one of claims 1 to 7, wherein in the treatment, brincidofovir, a pharmaceutically acceptable salt thereof, or a solvate thereof is administered at a dose of 10-90 mg.

9. The pharmaceutical composition according to any one of claims 1 to 8, wherein in the treatment, brincidofovir, a pharmaceutically acceptable salt thereof, or a solvate thereof is administered at a dose of 10-60 mg.

10. The pharmaceutical composition according to any one of claims 1 to 9, wherein in the treatment, brincidofovir, a pharmaceutically acceptable salt thereof, or a solvate thereof is administered at a dose of 30-60 mg.

11. The pharmaceutical composition according to claim 5 or 6, wherein in the treatment, brincidofovir, a pharmaceutically acceptable salt thereof, or a solvate thereof is administered at a dose of 15-30 mg.

12. A pharmaceutical composition for treatment of glioma, comprising temozolomide, a pharmaceutically acceptable salt thereof, or a solvate thereof, wherein in the treatment, (a) brincidofovir, a pharmaceutically acceptable salt thereof, or a solvate thereof, and (b) temozolomide, a pharmaceutically acceptable salt thereof, or a solvate thereof are used in combination.

13. The pharmaceutical composition according to claim 12, wherein in the treatment, radiation therapy is further used in combination.

14. A pharmaceutical composition comprising brincidofovir, a pharmaceutically acceptable salt thereof, or a solvate thereof, and temozolomide, a pharmaceutically acceptable salt thereof, or a solvate thereof.

15. A kit comprising brincidofovir, a pharmaceutically acceptable salt thereof, or a solvate thereof, and temozolomide, a pharmaceutically acceptable salt thereof, or a solvate thereof.
